# EUROPÄISCHE PATENTSCHRIFT

(11) **EP 3 577 521 B1**
(45) Veröffentlichungstag und Bekanntmachung des Hinweises auf die Patenterteilung: **10.03.2021**
(21) Anmeldenummer: 18702957.4
(22) Anmeldetag: 29.01.2018
(51) Int. Cl.: G03F 7/00, G03F 7/20, G03F 7/027, G03F 7/075, G03F 7/038, B33Y 70/00, B33Y 80/00

(54) **STRUKTURIERTES KOMPOSIT AUS MATRIXMATERIAL UND NANOPARTIKELN**
STRUCTURED COMPOSITE OF MATRIX MATERIAL AND NANOPARTICLES
COMPOSITE STRUCTURÉ COMPOSÉ D'UN MATÉRIAU DE MATRICE ET DE NANOPARTICULES

(30) Priorität: 31.01.2017 DE 102017101823
(43) Veröffentlichungstag der Anmeldung: 11.12.2019
(73) Patentinhaber: Fraunhofer-Gesellschaft zur Förderung der angewandten Forschung e.V., 80686 München (DE)
(72) Erfinder: HASSELMANN, Sebastian, 97082 Würzburg (DE); HEINRICH, Doris, 63975 Mespelbrunn (DE)
(74) Vertreter: Strehl Schübel-Hopf & Partner
(86) Internationale Anmeldenummer: PCT/EP2018/052026
(87) Internationale Veröffentlichungsnummer: WO 2018/141657

(56) Entgegenhaltungen:
- WO-A2-2014/108538
- BAOHUA JIA ET AL: "Highly Non-Linear Quantum Dot Doped Nanocomposites for Functional Three-Dimensional Structures Generated by Two-Photon Polymerization", ADVANCED MATERIALS, Bd. 22, Nr. 22, 11. Juni 2010 (2010-06-11) , Seiten 2463-2467, XP055083867, ISSN: 0935-9648, DOI: 10.1002/adma.201000513
- R. HOUBERTZ ET AL: "Investigations on the generation of photonic crystals using two-photon polymerization (2PP) of inorganic-organic hybrid polymers with ultra-short laser pulses", PHYSICA STATUS SOLIDI. A: APPLICATIONS AND MATERIALS SCIENCE, Bd. 204, Nr. 11, 1. November 2007 (2007-11-01), Seiten 3662-3675, XP055468512, DE ISSN: 1862-6300, DOI: 10.1002/pssa.200776416
- BAOHUA JIA ET AL: "Functional three-dimensional nonlinear nanostructures in a gold ion nanocomposite", QUANTUM ELECTRONICS CONFERENCE&LASERS AND ELECTRO-OPTICS (CLEO/IQEC/PACIFIC RIM), 2011, IEEE, 28. August 2011 (2011-08-28), Seiten 214-215, XP032175279, DOI: 10.1109/IQEC-CLEO.2011.6193776 ISBN: 978-1-4577-1939-4
- ANDREY L STEPANOV ET AL: "Synthesis and optical properties of silver nanoparticles in ORMOCER", APPLIED PHYSICS A; MATERIALS SCIENCE & PROCESSING, SPRINGER, BERLIN, DE, Bd. 108, Nr. 2, 28. März 2012 (2012-03-28) , Seiten 375-378, XP035086782, ISSN: 1432-0630, DOI: 10.1007/S00339-012-6894-6
- RUTH HOUBERTZ ET AL: "Advanced packaging materials for optical applications: bridging the gap between nm-size structures and large-area panel processing", PROCEEDINGS OPTICAL DIAGNOSTICS OF LIVING CELLS II, Bd. 6126, 9. Februar 2006 (2006-02-09), Seite 612605, XP055468885, US ISSN: 0277-786X, DOI: 10.1117/12.660140 ISBN: 978-1-5106-1723-0

## Beschreibung

### Gebiet der Erfindung

Die vorliegende Erfindung betrifft ein Komposit aus strukturiertem Matrixmaterial und Nanopartikeln, ein Verfahren zur Herstellung des Komposits sowie die Verwendung des Komposits.

### Stand der Technik

Im Bereich der Zellkultur, des Tissue Engineering und Lab-on-a-Chip-Systemen benötigt man Gerüststrukturen (Scaffolds), um die natürliche Umgebung von biologischen Zellen zu imitieren und über die Umgebung das Zellverhalten wie Proliferation, Adhäsion oder Differenzierung gezielt zu steuern. Durch Variation der Oberflächenrauigkeit, der Strukturierung im Mikrometerbereich oder der chemischen Oberflächenfunktionalisierung ist es beispielsweise möglich, das Verhalten biologischer Zellen zu beeinflussen. Bisher ist es nicht möglich, Scaffolds mit Größen im Zentimeterbereich mit einer Auflösung bis zur Nanometerskala innerhalb einer Probe herzustellen. Dies ist insbesondere nicht möglich in Kombination mit einer beliebigen dreidimensionalen Strukturierbarkeit. Zudem ist es für Biomaterialien von großem Vorteil, die Kontaktoberfläche und Oberflächenrauigkeit zu erhöhen ohne dabei die makroskalige dreidimensionale Struktur zu ändern.

Es gibt im Stand der Technik zahlreiche Materialien, die als biologische Gerüststruktur eingesetzt werden können, z. B. Hydrogele, Glas oder Fasern. Teilweise ist es auch möglich, diese mittels 3D-Druck-, Lithographie- oder MPP-Verfahren herzustellen. Das Polymerisationsverfahren mittels Mehr-Photonen-Polymerisation (MPP) oder Zwei-Photonen-Polymerisation (2PP) wird mittlerweile in vielen Bereichen eingesetzt.

Mesoporöse Nanopartikel können als Drug-Delivery System verwendet werden. TiO₂ Partikel werden verwendet, um die Oberflächenrauigkeit einzustellen, was allerdings ohne Matrix und ohne 3D-Strukturierung erfolgt.

DE 10 2007 020 302 B4 beschreibt 3D-Zellkulturen, in die verschiedenste Nanopartikel eingebettet werden. Diese können funktionalisiert sein oder verzögert freigesetzt werden. Strukturen sollen über ein kontaktloses Printverfahren, Elektrospinning- oder LIFT-Verfahren hergestellt werden.

WO 2012/041519 A2 offenbart ein Material, das biokompatibel und biofunktionalisiert ist, aus dem 2D- und 3D-Strukturen für die Medizintechnik hergestellt werden können. Zudem ist es photostrukturierbar (SL, 3D-Druck oder MPP). In das Polymer können biofunktionale

Gruppen (Wachstumsfaktoren, Antikörper, Peptide etc.) eingebracht/angebunden werden. In einer Ausführungsform werden die biofunktionellen Komponenten über Nanopartikel kovalent oder nicht kovalent an die Matrix gebunden. Die Nanopartikel können Hohlräume aufweisen, die mit Wirkstoff gefüllt sind und freigesetzt werden können.

US 2014/0080061 A1 offenbart ein Kompositmaterial, das mittels TPA vernetzbar ist und funktionalisierte Nanopartikel (auch Silica) enthält. Durch die Funktionalisierung können die Partikel kovalent an die Matrix gebunden werden

BAOHUA JIA ET AL, "Highly Non-Linear Quantum Dot Doped Nanocomposites for Functional Three-Dimensional Structures Generated by Two-Photon Polymerization", ADVANCED MATERIALS, Bd. 22, Nr. 22, 11. Juni 2010 (2010-06-11), Seiten 2463-2467 offenbart unter anderem die Herstellung von PbS dotierten nicht-linearen 3-dimensionalen Gerüststrukturen aus Ormocer-Resin, welche hierarchisch aufgebaut sind und zwei Strutureinheiten enthalten, wobei die eine Struktureinheit von der anderen Struktureinheit abzweigt und weniger als die Hälfte der Dicke anderen Struktureinheit hat. Die Gerüststrukturen werden mittels 2-Photonen-Polymerisation hergestellt.

### Durch die Erfindung zu lösende Aufgaben

Im Stand der Technik ist es daher nicht möglich, Scaffolds mit Größen im Zentimeterbereich mit einer Auflösung bis zur Nanometerskala innerhalb einer Probe herzustellen.

Dies ist insbesondere nicht möglich in Kombination mit einer beliebigen dreidimensionalen Strukturierbarkeit und einer Erhöhung der Kontaktoberfläche und der Oberflächenrauigkeit ohne gleichzeitige Änderung der makroskaligen dreidimensionalen Struktur.

Genauer gesagt offenbart der Stand der Technik nicht die Kombination von hierarchischem Aufbau der Strukturen mit hoher Genauigkeit und gleichzeitigem Einbau von Nanopartikeln in die Strukturen, sowie die gleichzeitige Einstellung der Oberflächenrauigkeit, insbesondere auf einen hohen Wert, durch Zugabe von Nanopartikeln.

Daher ist es die Aufgabe der vorliegenden Erfindung, ein verbessertes und einfaches Verfahren zur Herstellung von Kompositen bereitzustellen, die stukturiertes Material und Nanopartikel enthalten.

### Zusammenfassende Darstellung der Erfindung

Die Aufgabe wurde durch Bereitstellen eines Komposits gelöst, das photostrukturiertes Matrixmaterial und darin enthaltene Nanopartikel umfasst, wobei der Brechungsindex des strukturierten Materials und der Brechungsindex der Nanopartikel aufeinander abgestimmt sind. Ein weiterer Aspekt der Erfindung ist ein verbessertes Verfahren zur Herstellung eines Komposits sowie die Verwendung des Komposits für medizinische oder biologische Zwecke.

Der Gegenstand der vorliegenden Erfindung ist in den Ansprüchen definiert.

### Vorteile der Erfindung

Die Vorteile des erfindungsgemäßen Verfahrens sind die Bereitstellung einer hierarchischen Struktur mit hoher Genauigkeit unter gleichzeitigem Einbau von Nanopartikeln. Insbesondere kann durch das erfindungsgemäße Verfahren eine höhere Konzentration von Nanopartikeln unter Beibehaltung der Genauigkeit der Struktur eingesetzt werden.

Bezüglich der hierarchischen Struktur erlaubt das erfindungsgemäße Verfahren, verschiedenste Photostrukturierungsverfahren innerhalb einer Probe zu verbinden und somit Strukturen im Zentimetermaßstab (z.B. 3D-Druck) mit einer (Sub-) Mikrometerstruktur (MPP bzw. 2PP) zu versehen. Die Struktur weist zusätzlich durch Nanopartikel eine intrinsische Nanostrukturierung der Oberfläche auf. Durch die Wahl der 3D-Scaffold-Geometrien und der Partikelgrößen lassen sich beliebige 3D-Scaffolds oder Strukturen erzeugen. Durch die Auswahl der Nanopartikel und des photostrukturierbaren Materials kann eine Zusammensetzung für das Photostrukturierungsverfahren bereitgestellt werden, in der die Brechungsindices der Komponenten so eingestellt sind, insbesondere auf einen so ähnlichen Wert eingestellt sind, dass die gewünschte Genauigkeit der Photostrukturierung ermöglicht wird.

Durch den Aufbau des Komposits als hierarchische Struktur lassen sich Systeme bereitstellen, die einerseits sehr stabil sind und andererseits eine hohe innere Oberfläche aufweisen. Zudem lassen sich durch die Kombination von verschiedenen Strukturierungsverfahren Proben deutlich zeit- und kosteneffizienter herstellen.

Bezüglich der einstellbaren Oberflächenrauigkeit lassen sich über die Wahl der Nanopartikel (Größe, Material, Porosität und dergleichen) zusätzlich verschiedenste intrinsische Eigenschaften der 2D-/3D-Strukturen steuern, beispielsweise solche, mit denen das Zellverhalten beeinflusst werden kann. Dazu zählen die Oberflächenrauigkeit, Wirkstofffreisetzung, Oberflächenchemie, Antimikrobiell und dergleichen. In Kombination mit mikrofluidischen Systemen lassen sich hiermit Lab-on-a-Chip bzw. Organ-on-a-Chip Systeme mit maßgeschneiderten Eigenschaften für verschiedenste Anwendungen herstellen. Bei Verwendung einer bioabbaubaren Matrix lassen sich ebenfalls 3D-Scaffolds für Implantate herstellen, die unter Verwendung von wirkstoffgeladenen Partikel auch als Drug-Delivery System genutzt werden können.

Bezüglich der hohen Oberflächenrauigkeit können die Nanopartikel und das photostrukturierbare Material so ausgewählt werden, dass sich die Brechungsindices nur geringfügig unterscheiden. Dadurch ist eine hohe Genauigkeit der Photostrukturierung möglich. Durch die Einstellung der Brechungsindices ist es möglich, eine hohe Konzentration von Nanopartikeln einzusetzen und trotzdem eine hohe Genauigkeit der Photostrukturierung zu erreichen.

Durch die Einstellung bzw. Anpassung der Brechungsindices wird eine größere Bandbreite an Kombinationen von photostrukturiertem Material und durch Nanopartikel verliehene Eigenschaften ermöglicht.

Ein beispielhaftes erfindungsgemäßes Komposit, das Silica, ORMOCER® I enthält (vgl. Tabelle 3), zeigte sich in einem Versuch nach ISO 10993 als nicht zytotoxisch, so dass sie aufgrund dieser Biokompatibilität für den Einsatz als Medizinprodukt geeignet sind.

Ein weiteres Kriterium für den Einsatz im Bereich der Medizintechnik, insbesondere bei Implantaten, ist die Sterilisierbarkeit. Das in der vorliegenden Erfindung beispielhaft eingesetzte Komposit kann ohne strukturelle oder topographische Veränderungen autoklaviert werden. Neben dieser Sterilisationsmöglichkeit können die Kompositsysteme außerdem mit einem 70% Ethanol/Wasser-Gemisch oder durch Gamma-/ UV-Bestrahlung behandelt werden.

Es zeigte sich außerdem, dass durch die verwendete Nanopartikelgröße, die Oberflächentopographie und die Oberflächenstrukturierung im Mikrometerbereich durch TPA das Zellverhalten beeinflusst werden kann.

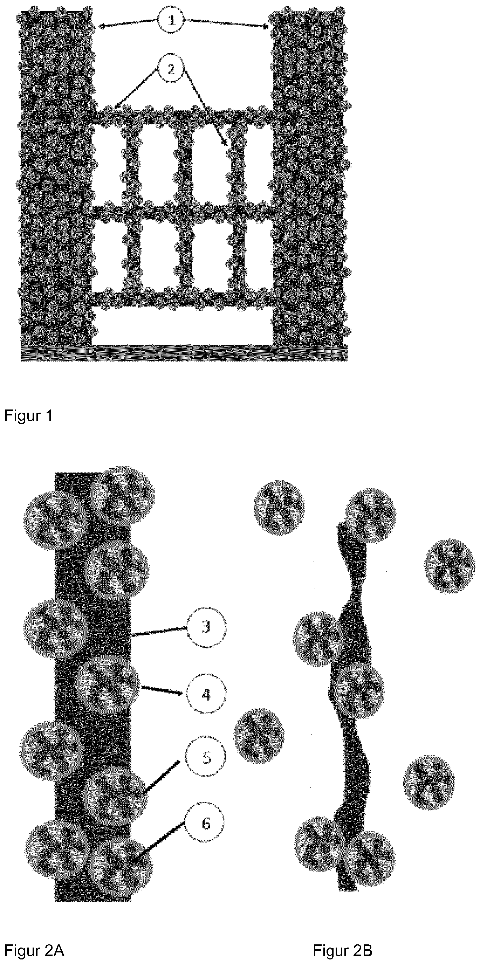

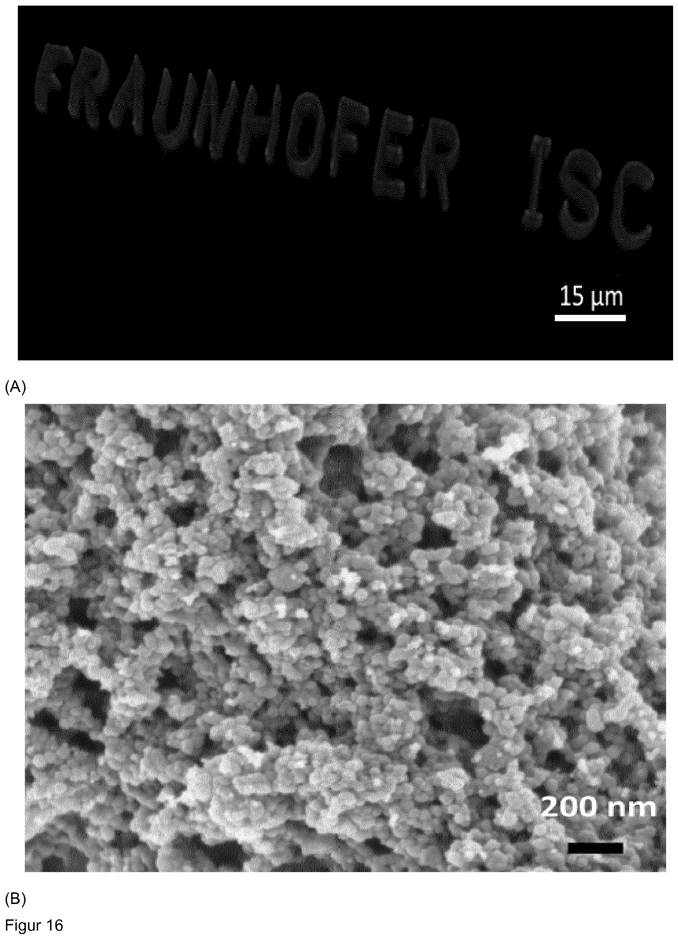

Bei der Verwendung von hohlen Partikeln innerhalb einer biodegradierbaren Matrix kann durch die Verwendung von Nährstoffen, Wachstumsfaktoren oder andere Wirkstoffe gezielt das Zellverhalten beeinflusst werden. Dabei wird der Wirkstoff während des Abbaus der Matrix allmählich freigesetzt. Dieses Konzept ist schematisch in Figur 2 gezeigt und ausführlich in den Beispielen und Figur 16 beschrieben.

### Beschreibung der Figuren

Figur 1 zeigt einen schematischen Querschnitt eines möglichen 3D-Scaffold-Designs, das durch Kombination mehrerer photostrukturierbarer Verfahren aufgebaut wurde. Dadurch ist eine hierarchische Struktur vom Zentimeter- bis in den Nanometerbereich beliebig anpassbar.
Figur 2 zeigt in (a) eine schematische Darstellung eines Komposits mit den einzelnen Komponenten, wobei die Matrix aus ORMOCER® aufgebaut ist. Die schematische Darstellung der Freisetzung der wirkstoffgeladenen mesoporösen Nanopartikel während des biologischen Abbaus der biokompatiblen Matrix ist in (b) gezeigt.
Figur 3A) zeigt eine REM Aufnahme einer mittels 2PP hergestellten 3D-Struktur aus Komposit OC-E. Die Skala entspricht 4 µm.
Figur 3B) zeigt eine Zoom-Aufnahme des Ausschnitts aus Figur 3A), die die Nanopartikel und die entsprechende Oberflächenrauigkeit der 3D-Struktur zeigt. Die Skala entspricht 400 nm.
Figur 4 zeigt eine REM-Aufnahme von 3D-Strukturen, die mittels MPP-Verfahren hergestellt wurden. Für die Struktur in A) wurde OC-D verwendet; die Skala entspricht 2 µm. Für die Struktur in B) wurde OC-B verwendet; die Skala entspricht 10 µm.
Figur 5 zeigt eine REM-Aufnahme einer mittels MPP hergestellten Struktur aus Komposit OC-D, die zwischen zwei lithographisch hergestellten Quadern aus ORMOCER® angeordnet ist. A) Aufsicht; B) um 60° gekippte Aufnahme, die den Übergang zwischen lithographisch und mittels MPP hergestellten Strukturen zeigt. Die Skala in A) entspricht 20 µm, die Skala in B) entspricht 3 µm.
Figur 6 zeigt den Zusammenhang zwischen dem Brechungsindex und den Gewichtsanteilen von ORMOCER® I und ORMOCER® II in einem Gemisch.
Figur 7 zeigt die anzahlgewichtete Partikelgrößenverteilung der in den Beispielen verwendeten Silica-Nanopartikel, die rasterelektronenmikroskopisch ermittelt wurden.
Figur 8 zeigt rasterkraftmikroskopische Aufnahmen der gerakelten Oberflächen, ohne Füllstoff (OC-A) und gefüllt mit zunehmendem Partikeldurchmesser (OC-B bis OC-D). Die Skala entspricht 2 µm.
Figur 9 zeigt eine Migrationsanalyse mit Zellen von D. Dictyostelium auf gerakelten Oberflächen, ohne Füllstoff (OC-A) und gefüllt mit zunehmendem Partikeldurchmesser (OC-B bis OC-D). Aktive Migrationsphasen sind heller und passive Migrationsphasen dunkler dargestellt.
Figur 10A) zeigt eine REM-Aufnahme einer mittels MPP hergestellten 3D-Struktur aus einem Komposit, bestehend aus superparamagnetischen Magnetitpartikeln und ORMOCER®. Figur 10B) zeigt die Vergrößerung des in Figur 10A) markierten Ausschnitts. Die Skala in A) entspricht 2 µm, die Skala in B) entspricht 200 nm.
Figur 11: Mittels WST-Test bestimmte Zellviabilität muriner L929 Fibroblasten, die 48 Stunden in einem Extrakt kultiviert wurden. Das Extrakt wurde durch Inkubation der zu untersuchenden Proben in DMEM-Medium für verschiedene Zeitdauern hergestellt.
Inkubiertes Medium ohne Probe dient als Referenz und entspricht einer Viabilität von 100%.
Figur 12: Rasterlasermikroskopaufnahmen des gekreuzten Linienfelddesigns für Untersuchungen der Autoklavierbarkeit und Zellmorphologie am Beispiel von OC-D. Die Linien haben einen quadratischen Querschnitt von 5x5 µm und haben eine Gesamtlänge von 1,5 mm. (A): Aufsicht der Gesamtstruktur. (B): 3D Ansicht eines Ausschnitts des Bereichs mit gekreuzten Linien.
Figur 13: Rasterelektronenmikrokopische Aufnahmen von gekreuzten Linienstrukturen vor dem Autoklavieren (A) und nach fünfmaligem Autoklavieren (B) für verschiedene Kompositsysteme: A) OC-A, B) OC-B, C) OC-D.
Figur 14: (A): Rasterelektronenmikroskopaufnahme des Probenbereichs mit gekreuzten Linien am Beispiel von OC-D. (B): 3D Fluoreszenzmikroskopaufnahme von 3T3-Mausfibroblasten nach 48 Stunden Inkubation auf der Struktur.
Figur 15: Sphärizität und Elongation von 3T3-Zellen auf unstrukturiertem (flach) und mittels TPA strukturiertem Substrat (parallele und gekreuzte Linien) aus den Kompositen OC-A, OC-B und OC-D basierend auf dreidimensionalen Fluoreszenzaufnahmen.
Figur 16: Mittels TPA strukturiertes Komposit, bestehend aus einer partiell biodegradierbaren ORMOCER®-Matrix und mesoporösen Silicapartikeln, die mit einem Fluoreszenzfarbstoff gefüllt und mit Polyvinylpyrrolidon ummantelt sind. (A): dreidimensionale Fluoreszenzmikroskopaufnahme. (B): Rasterelektronenmikroskopaufnahme der Oberfläche.

### Ausführungsformen der Erfindung

Der hier verwendete Ausdruck "photostrukturierbares Material" oder "photostrukturierbares Matrixmaterial" umfasst jedes Material oder jede Zusammensetzung unterschiedlicher Materialien, das bzw. die durch Behandlung mit elektromagnetischer Strahlung polymerisiert werden kann. Die elektromagnetische Strahlung kann beispielsweise durch ein fokussiertes Licht oder durch einen Laserstrahl gebildet sein. Das Material kann ein Vorläufer eines Polymers sein, beispielsweise ein Monomer. Ein Beispiel eines photostrukturierbaren Materials ist ein ORMOCER®, d.h. eine hydrolytisch kondensierte Silan-Verbindung, die organisch vernetzbare Gruppen trägt.

Das photostrukurierbare Material kann unterschiedliche photostrukturierbare Materialen enthalten. Das kann einerseits bedeuten, dass zwei oder mehr Materialien unterschiedlicher Struktur, beispielsweise unterschiedliche Polymervorläufer, als Gemisch enthalten sein können. Andererseits kann es auch bedeuten, dass es sich um unterschiedliche photostrukturierbare Gruppen in ein und derselben Verbindung handelt, beispielsweise um reaktive Gruppen unterschiedlicher Struktur und Photoreaktivität in einem einzigen Polymervorläufermolekül.

Im Zusammenhang mit der vorliegenden Erfindung bedeutet "Gerüststruktur" oder "Scaffold" vorzugsweise eine dreidimensionale Matrix, die vorzugsweise Poren oder Zwischenräume aufweist. Die Struktur kann zum Anheften von Zellen eingesetzt werden. Eine Gerüststruktur im Rahmen der vorliegenden Erfindung ist insbesondere in bevorzugter Ausführung eine Struktur, die nicht allein ein oberflächliches Anhaften oder Adhärieren von Zellen gestattet, sondern darüber hinaus insbesondere auch ein Hineinwachsen oder Integrieren von Zellen in die Gerüststruktur selbst.

In der vorliegenden Erfindung werden Strukturen oder dreidimensionale Gerüststrukturen unterschiedlicher Größenbereiche gebildet. Beispielsweise werden Strukturen im Millimeter-, Mikrometer-, Submikrometer- oder Nanometerbereich hergestellt. Dabei bedeutet die Angabe eines Größenwertes einer Struktur, dass in der Struktur Freiräume, Poren oder Hohlräume entstehen, die zumindest in einer Raumrichtung, also in Länge, Breite oder Tiefe, die Abmessungen des angegebenen Wertes aufweisen. Vorzugsweise entsteht eine Struktur mit Poren, deren Ausmaße diesem Größenwert entsprechen. Wenn beispielsweise eine Struktur im Bereich von 1 bis 100 µm gebildet wird, heißt das, dass Poren einer Größe von etwa 1 bis 100 µm entstehen. Entsprechendes gilt für die anderen hier verwendeten Angaben von Größenwerten für Strukturen.

Das erfindungsgemäße Komposit ist hierarchisch aufgebaut und weist beispielsweise folgende drei Hierarchieebenen auf:
Ebene 1: eine Überstruktur einer Größe von 10 µm bis 100 mm, vorzugsweise 100 µm bis 50 mm, stärker bevorzugt 500 µm bis 5 mm;
Ebene 2: eine Feinstruktur einer Größe von 100 nm bis 1000 µm, vorzugsweise 1000 nm bis 100 µm, stärker bevorzugt 5 µm bis 50 µm;
Ebene 3: Nanopartikel einer Größe von 1 bis 1000 nm, vorzugsweise 5 bis 800 nm, stärker bevorzugt 10 bis 500 nm.

In jedem Fall weist die jeweilige Überstruktur so große Freiräume oder Poren auf, dass eine Vielzahl von gewünschten Feinstrukturen darin gebildet werden kann. Und in jedem Fall weist die jeweilige Feinstruktur so große Freiräume oder Poren auf, dass eine Vielzahl von eingesetzten Nanopartikeln darin enthalten sein kann. Der Ausdruck "Vielzahl" bedeutet mindestens 5, mindestens 10, mindestens 50 oder mindestens 100 oder 5 bis 1000, vorzugsweise 10 bis 500, stärker bevorzugt 20 bis 300 und am stärksten bevorzugt 30 bis 100. Anders ausgedrückt bedeutet Vielzahl, dass die Struktur in mindestens einer Raumrichtung, vorzugsweise in mindestens zwei Raumrichtungen, also in Längen-, Breiten- oder Tiefenrichtung, ein Ausmaß aufweist, das um den Faktor 5 bis 1000, vorzugweise 5 bis 100 oder 10 bis 1000, stärker bevorzugt 5 bis 50 oder 10 bis 100 größer ist als die mittlere Größe der Nanopartikel.

Das erfindungsgemäße Komposit ist hierarchisch aufgebaut. Das heißt, es weist dickere und weniger dicke Struktureinheiten auf. Die weniger dicke, also die dünnere Struktureinheit zweigt von der dickeren Struktureinheit ab. Diese Verbindungsstelle, also die Abzweigung, kann einen beliebigen Winkel annehmen, ist aber bevorzugt 90 °, so dass eine T-förmige Struktur entsteht. Eine solche Struktureinheit ist ein beliebig großer Bereich einer Struktur, ist jedoch vorzugsweise die gesamte Struktur, die bei einer Abzweigung beginnt und, falls vorhanden, bei der nächsten Abzweigung endet, also der Abschnitt zwischen zwei Abzweigungen. In einem Raumsystem mit den Koordinaten in x-, y- und z-Richtung ist die Dicke einer gegebenen Struktureinheit in einem dreidimensionalen Kompositsystem definiert als der kleinste Wert der Werte von x, y und z. Bei einer flächigen Struktureinheit mit einer Länge x und einer Breite y ist die Dicke folglich das Ausmaß in z-Richtung. Wenn eine Struktureinheit von einer anderen abzweigt, gilt für jede Struktureinheit unabhängig von der anderen Struktureinheit ein eigenes Raumsystem mit x-, y- oder z-Richtung. Das heißt, dass bei zwei Struktureinheiten die Dickenrichtung der ersten Struktureinheit nicht die gleiche Richtung im Raum haben muss wie die Dickenrichtung der zweiten Struktureinheit. Die hierarchische Anordnung mit den Abzweigungen schließt natürlich andere Strukturelemente, beispielsweise eine Abzweigung oder Kreuzung von Struktureinheiten gleicher Hierarchiestufe, nicht aus. Die Ausmaße der Struktureinheiten werden vorzugsweise ohne Berücksichtigung der ggf. vorhandenen und aus der Struktureinheit herausragenden Nanopartikel ermittelt.

Die Ausmaße der jeweiligen Strukturbereiche können durch entsprechende Anpassung der Verfahrensparameter beim Photostrukturieren eingestellt werden. In einem gegebenen Komposit können die Ausmaße mittels Rasterelektronenmikroskopie ermittelt werden.

Beispiele für das Durchführen von hierarchischem Strukturieren sind in US 2003/0013047 A1 offenbart, wo beispielsweise in Abschnitt [0012] ein Verfahren beschrieben wir, bei dem zuerst eine Grobprozessierung mit Ein-Photonen-Polymerisation und anschließend eine Feinprozessierung mit Zwei-Photonen-Polymerisation beschrieben erfolgt.

In dem erfindungsgemäßen Komposit ist die hierarchische Struktur durch mindestens zwei Hierarchiestufen definiert. Eine Struktureinheit (I) stellt die übergeordnete Hierarchiestufe dar, Struktureinheit (II) folgt als nächste Hierarchiestufe, und Struktureinheit (III) stellt die unterste der drei Stufen dar. Die Anzahl der Hierarchiestufen ist natürlich nicht auf drei begrenzt. Die durch die Nanopartikel erzeugte zusätzliche Oberfläche stellt eine weitere Hierarchiestufe dar.

In der Ausführungsform (A1) mit mindestens zwei Hierarchiestufen enthält das Komposit mindestens eine Struktureinheit (I) einer aus dem Bereich von 10 µm bis 100 mm ausgewählten Dicke (i) und von der Struktureinheit (I) abzweigende Struktureinheiten (II) einer jeweils aus dem Bereich von 100 nm bis 1000 µm ausgewählten Dicke (ii), wobei an den Abzweigungen die Dicke (ii) höchstens die Hälfte der Dicke (i) ist.

In einer Ausführungsform (A2) mit mindestens drei Hierarchiestufen enthält das Komposit mindestens eine Struktureinheit (I) einer aus dem Bereich von 100 µm bis 100 mm ausgewählten Dicke (i), von der Struktureinheit (I) abzweigende Struktureinheiten (II) einer jeweils aus dem Bereich von 10 µm bis 1000 µm ausgewählten Dicke (ii) sowie von den Struktureinheiten (II) abzweigende Struktureinheiten (III) einer jeweils aus dem Bereich von 100 nm bis 100 µm ausgewählten Dicke (iii), wobei an den Abzweigungen der Struktureinheiten (II) von der Struktureinheit (I) die Dicke (ii) höchstens die Hälfte der Dicke (i) ist und an den Abzweigungen der Struktureinheiten (III) von den Struktureinheiten (II) die Dicke (iii) höchstens die Hälfte der Dicke (ii) ist.

Die Dicke der Struktureinheit einer unteren Hierarchiestufe ist höchstens der Hälfte der Dicke der jeweils übergeordneten Hierarchiestufe, also (iii) im Verhältnis zu (ii) und (ii) im

Verhältnis zu (i), in weiteren Ausführungsformen höchstens ein Drittel, höchstens ein Viertel oder höchstens ein Zehntel. Diese Dickenverhältnisse gelten für die Abzweigungsstellen, vorzugsweise aber auch für die restlichen Bereiche der jeweiligen Struktureinheiten.

In Ausführungsform (A1) ist der Bereich für die Dicke (i) bevorzugt 100 µm bis 10 mm, stärker bevorzugt 100 µm bis 5 mm, und der Bereich für die Dicke (ii) bevorzugt 1000 nm bis 100 µm, stärker bevorzugt 1000 nm bis 50 µm. Bevorzugt ist eine Ausführungsform (A1) mit der Kombination der jeweils genannten bevorzugten Bereiche, stärker bevorzugt ist eine Ausführungsform (A1) mit der Kombination der jeweils genannten stärker bevorzugten Bereiche.

In Ausführungsform (A2) ist der Bereich für die Dicke (i) bevorzugt 500 µm bis 10 mm, stärker bevorzugt 1000 µm bis 5 mm, der Bereich für die Dicke (ii) bevorzugt 50 bis 500 µm, stärker bevorzugt 100 bis 500 µm, und der Bereich für die Dicke (iii) bevorzugt 1000 nm bis 100 µm, stärker bevorzugt 1000 nm bis 50 µm. Bevorzugt ist eine Ausführungsform (A2) mit der Kombination der jeweils genannten bevorzugten Bereiche, stärker bevorzugt ist eine Ausführungsform (A2) mit der Kombination der jeweils genannten stärker bevorzugten Bereiche.

Die Struktureinheiten (I), (II) und (III) können mit den gleichen oder mit jeweils verschiedenen Strukturierungsverfahren hergestellt werden. Grundsätzlich kann für jede Strukturhierarchiestufe jedes bekannte Strukturierungsverfahren eingesetzt werden. Einschränkungen ergeben sich für den Fachmann ersichtlich jedoch dadurch, dass für bestimmte Dicken von Struktureinheiten bestimmte Strukturierungsverfahren besser oder einfacher durchzuführen oder nur bestimmte Strukturierungsverfahren überhaupt geeignet sind.

Durch die Kombination verschiedener photostrukturierender Verfahren können gewünschte hierarchische Strukturen aufgebaut werden. Auf diese Weise ist eine hierarchische Struktur vom Zentimeter- bis zum Nanometerbereich beliebig anpassbar. Beispielsweise können Überstrukturen im Zentimeterbereich durch Rapid-prototyping oder 3D-Druck, Strukturen im Millimeter- bis Mikrometerbereich durch Lithographie und Strukturen im Submikrometerbereich durch MPP realisiert werden.

Das erfindungsgemäße Verfahren umfasst die vorstehend beschriebenen Stufen (a) und (b). Die in Stufe (b) jeweils eingesetzten Polymerisationsreaktionen zur Bildung der Struktureinheiten (I) und (II) bzw. (I), (II) und (III) können gleichzeitig oder nacheinander durchgeführt werden. Wenn sie gleichzeitig durchgeführt werden, verbinden sich die jeweiligen Struktureinheiten an den Grenzflächen durch die gleichzeitige Polymerisation der Ausgangsmaterialien. Wenn sie nacheinander durchgeführt werden, verbinden sich die jeweiligen Struktureinheiten an den Grenzflächen ebenfalls, da die Polymerisation durch Auswahl der Ausgangsmaterialien und Verfahrensparameter so durchgeführt werden kann, dass eine unvollständige Vernetzung erfolgt. Beispiele für typische Vernetzungsgrade sind im Bereich von 60 bis 80 %. Die nicht umgesetzten reaktiven Gruppen können dann bei der nachfolgenden Polymerisation einer weiteren Struktureinheit zur kovalenten Verknüpfung der neu polymerisierten mit der schon vorhandenen Struktureinheit verwendet werden. Es kann insbesondere dann vorteilhaft sein, die Polymerisationen nacheinander durchzuführen, wenn für die Bildung einer Struktureinheit ein anderes Photostrukturierungsverfahren als für die Bildung einer weiteren Struktureinheit verwendet wird. Ein Beispiel ist die Bildung einer Struktureinheit (I) mittels 3D-Druck und die nachfolgende Bildung einer Struktureinheit (II) mittels MPP.

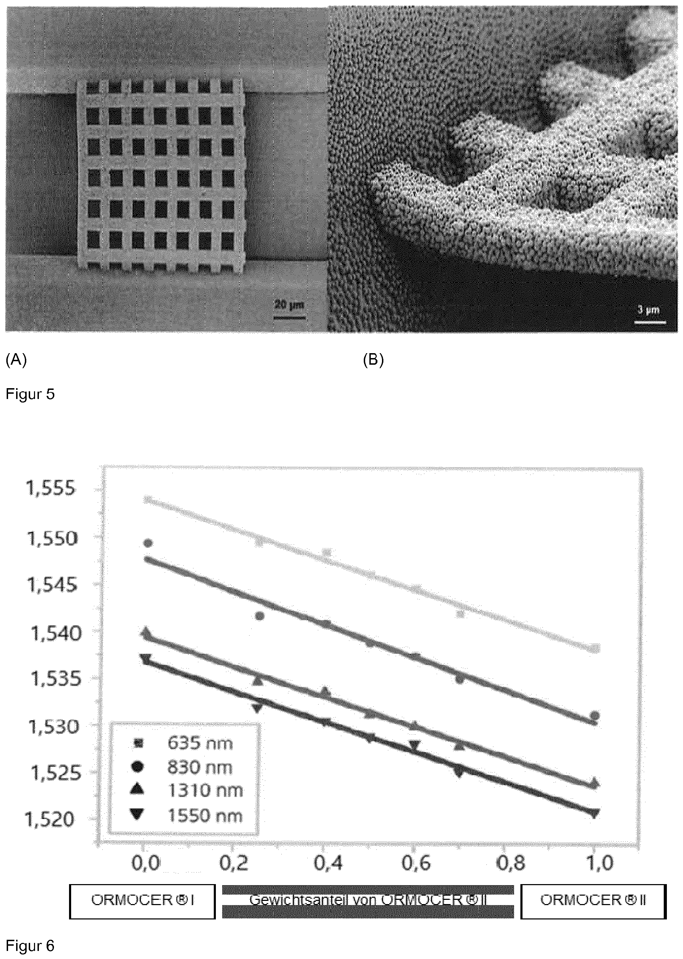

Aufeinanderfolgende Strukturierungsverfahren können auch eingesetzt werden, wenn eine Änderung der Ausgangsmaterialien und/oder die Verfahrensparameter erfolgen soll. Beispielsweise wird in einer Ausführungsform eine besonders stabile Struktureinheit (I) aus photostrukturierbarem Material ohne Zusatz von Nanopartikeln als übergeordnete Gerüststruktur hergestellt. Danach wird das nicht umgesetzte Material entfernt und ein Kompositansatz, der neben photostrukturierbarem Material auch Nanopartikel enthält, zu der Gerüststruktur gegeben. Dieser Ansatz kann dann unter Bildung von Struktureinheiten (II) und ggf. (III) und unter Einbindung von Nanopartikeln polymerisiert werden. In dieser Ausführungsform wird vor Stufe (a) eine Struktureinheit des erfindungsgemäßen Komposits hergestellt. Eine solche Ausführungsform zeigt Figur 5. In Figur 5(B) ist zu sehen, dass die ohne Nanopartikel hergestellte Struktureinheit durch die Verwendung von Nanopartikeln in der nachfolgenden Reaktion zumindest oberflächlich mit Nanopartikeln bedeckt wird.

In anderen Ausführungsformen des erfindungsgemäßen Komposits sind jedoch sowohl die Struktureinheiten (I) als auch die Struktureinheiten (II) sowie ggf. (III) aus dem Kompositansatz aus photostrukturierbarem Matrixmaterial und Nanopartikeln hergestellt worden bzw. herstellbar, so dass Nanopartikel in allen diesen Struktureinheiten enthalten sind.

Das erfindungsgemäße Komposit weist wegen der hierarchischen Struktur eine große innere Oberfläche auf. Die innere Oberfläche kann mit geeigneten mikroskopischen Verfahren durch Auswertungen von Querschnitten des Komposits ermittelt werden. Die Gesamtoberfläche des Komposits wird zusätzlich zu der großen inneren Oberfläche auch durch die große Oberflächenrauigkeit des Komposits aufgrund des Vorhandenseins der Nanopartikel erhöht. Durch entsprechende Auswahl der Messgenauigkeiten und der Auswerteparamater, insbesondere der Auflösung, kann mit den mikroskopischen Verfahren die innere Oberfläche des Komposits entweder unter Berücksichtigung oder ohne Berücksichtigung der zusätzlichen, von den Nanopartikeln herrührenden Oberfläche ermittelt werden. Wenn beispielsweise mit einer Auflösung von 1 µm ausgewertet wird, bleibt die von Nanopartikeln einer Größe von beispielsweise 100 nm herrührende zusätzliche Oberfläche unberücksichtigt. Die innere Oberfläche ohne Berücksichtigung der von den Nanopartikeln herrührenden zusätzlichen Oberfläche, also allein durch die Ausgestaltung des Komposits als hierarchische Struktur erhaltene innere Oberfläche ist bei einer Auflösung von 1 µm vorzugsweise mindestens doppelt so groß wie die äußere Oberfläche, stärker bevorzugt mindestens fünfmal so groß und noch stärker bevorzugt mindestens zehnmal so groß.

Im Zusammenhang mit der vorliegenden Erfindung bedeutet "dreidimensional" eine räumliche Ausdehnung in alle drei Raumkoordinaten. Die Ausdehnung kann im Wesentlichen gleichmäßig in diesen drei Richtungen vorliegen, sodass zum Beispiel eine zylindrische Form, eine säulen-, quader- oder würfelförmige Matrixstruktur vorliegt. Es ist aber zum Beispiel auch möglich, dass die Ausdehnung in zwei Richtungen in größerem Ausmaß vorliegt, in die dritte Richtung jedoch nur in geringem Maße, so dass die dreidimensionale Struktur flächig wirkt, zum Beispiel eine Membran oder Schicht darstellt. Für die einzelnen Struktureinheiten des Komposits ist eine solche Flächenform bevorzugt, das heißt, sie weisen eine Länge x, eine Breite y und eine Dicke z auf, wobei x und y jeweils mindestens doppelt, vorzugsweise mindestens dreimal und stärker bevorzugt mindestens fünfmal so groß wie z sind.

Im Rahmen der vorliegenden Erfindung bedeutet der Begriff "biokompatibel", dass die Gerüststruktur und die Nanopartikel sowohl hinsichtlich ihrer Materialzusammensetzung als auch hinsichtlich ihrer Struktur in den Zellen, Geweben oder in einem Organismus, insbesondere eines Versuchstiers, keine toxische, apoptotische, unerwünschte immunologische oder sonstige unerwünschte Reaktion hervorruft und zelluläre und molekulare Prozesse auch nach einer möglichen Internalisierung der Nanopartikel oder einem Abbau der Nanopartikel und/oder Gerüststruktur nicht oder kaum stört.

### Komposit aus Matrix und Nanopartikeln

In einer Ausführungsform der vorliegenden Erfindung ist es vorgesehen, dass die Matrix Nanopartikel enthält, die in der Matrix integral in der Gerüststruktur verteilt vorliegen. Die Nanopartikel können in bevorzugter Ausführung homogen in der Gerüststruktur verteilt vorliegen. Es kann in bevorzugter Ausführung aber auch eine heterogene, ungleichmäßige Verteilung der Nanopartikel vorliegen. Hierbei könnte die Partikelkonzentration als Gradient in der Gerüststruktur vorliegen. Sind die Partikel mit Wirkstoff gefüllt erreicht man ebenfalls einen Wirkstoffgradienten innerhalb der Gerüststruktur. In einer weiteren bevorzugten Ausführungsform liegen die Nanopartikel in Form mindestens einer Schicht ober- und/oder unterhalb der Gerüststruktur vor. In einer weiteren bevorzugten Ausführungsform liegen die Nanopartikel in Form mindestens einer Schicht oberhalb der Gerüststruktur vor. Vorzugsweise sind die Matrixmaterialien und/oder die Nanopartikel, stärker bevorzugt die Matrixmaterialien und die Nanopartikel, biokompatibel und/oder bioabbaubar, stärker bevorzugt biokompatibel und bioabbaubar.

In einer bevorzugten Ausführungsform der vorliegenden Erfindung ist der mittlere Durchmesser der Nanopartikel stets kleiner als oder gleich wie die mittlere Dicke der Gerüststruktur. In einer weiteren bevorzugten Ausführungsform ist der mittlere Durchmesser aller Nanopartikel stets kleiner als die mittlere Dicke der Gerüststruktur. In einer bevorzugten Ausführungsform liegen bevorzugt alle Nanopartikel, bevorzugt überwiegend oder vollständig, in der Gerüststruktur eingebettet vor. Bevorzugt beträgt das Verhältnis der Abmessungen von Nanopartikeln zur Dicke der Gerüststruktur 1:1 oder weniger, bevorzugt 1:10 oder weniger, weiter bevorzugt 1:100 oder weniger, weiter bevorzugt 1:1000 oder weniger.

Vorzugsweise sind die Nanopartikel in der Gerüststruktur in einem hohen Anteil enthalten. In dem Kompositansatz oder dem Komposit der Erfindung sind die Nanopartikel in einem Volumenanteil von mindestens 1%, können aber auch in einem Volumenanteil von mindestens 10% oder mindestens 20% enthalten sein. Bevorzugt sind Volumenanteile von 1 bis 60%, stärker bevorzugt 5 bis 30%, noch stärker bevorzugt 10 bis 30% und am stärksten bevorzugt 10 bis 20%. In einer Ausführungsform können die Nanopartikel in dem erfindungsgemäßen Komposit in einem Gewichtsanteil von mindestens 1%, mindestens 10% oder mindestens 20% enthalten sein. Bevorzugt sind Gewichtsanteile von 1 bis 50%, stärker bevorzugt 5 bis 40%, noch stärker bevorzugt 10 bis 30 % und am stärksten bevorzugt 10 bis 20%.

Die hier genannten Volumenanteile und Gewichtsanteile beziehen sich jeweils auf das Trockengewicht alle Komponenten.

Die Nanopartikel werden durch die Polymerisation des Matrixmaterials an die entsehende Matrix gebunden. Wenn das Polymer beispielsweise durch Zwei-Photonen-Absorption hergestellt wird, wird der Kompositansatz in einem definierten Bereich beleuchtet. In diesem Bereich ist die Lichtstärke im Fokus am stärksten und nimmt von Inneren des Fokus nach außen hin ab, so dass auch der Grad der Vernetzung des Polymers von innen nach außen abnimmt. Es entstehen also innen ein stark vernetztes Polymer, weiter außer ein schwach vernetztes Polymer und noch weiter außen verbleibt unvernetztes Polymer. Nanopartikel sind in diesem Stadium an das stark vernetzte und schwach vernetzte Polymer gebunden. Nach der Polymerisation erfolgt ein Entwicklungsvorgang, in dem durch die eingesetzten Chemikalien sowohl das schwach vernetzte als auch das unvernetzte Polymer entfernt werden. Als Komposit bleibt schließlich das stark vernetzte Polymer mit den daran gebundenen Nanopartikeln übrig. Da Nanopartikel auch an der Grenzfläche von stark und schwach vernetztem Polymer gebunden sind, ragt eine Anzahl von Nanopartikeln zumindest teilweise aus dem Komposit heraus.

Vorzugweise ist in dem erfindungsgemäßen Komposit die Oberflächenrauigkeit hoch. Die Oberflächenrauigkeit wird in der vorliegenden Erfindung mittels Rasterkraftmikroskopie bestimmt und als Wert Z_{z} in Nanometer angegeben. Bevorzugte Werte von Z_{Z} sind 10 bis 10000 nm oder 10 bis 1000 nm, vorzugsweise 30 bis 1000 nm, stärker bevorzugt 50 bis 800 nm und am stärksten bevorzugt 100 bis 800 nm. Alternativ kann die Oberflächenrauigkeit als Wert Zq angegeben werden. Bevorzugte Werte von Zq sind 1 bis 10000 nm, stärker bevorzugt 1 bis 1000 nm, noch stärker bevorzugt 1 bis 100 nm und besonders bevorzgut 1 bis 50 nm oder 50 bis 100 nm. Die Oberflächenrauigkeit wird bestimmt durch die Größe und die Anzahl oder den Volumenanteil der Nanopartikel in dem Komposit. Die bevorzugte Kombination aus mittlerer Größe der Nanopartikel und Volumenanteil der Nanopartikel in dem Komposit ist 5 bis 1000 nm bei 3 bis 50%, stärker bevorzugt 10 bis 1000 nm bei 5 bis 50%, noch stärker bevorzugt 30 bis 800 nm bei 10 bis 40%. Dabei ist bei größeren Nanopartikeln der Volumenanteil geringer, so dass es bevorzugt ist, dass das Produkt aus dem in Nanometer angegebenen Zahlenwert der mittleren Größe der Nanopartikel und dem in Prozent angegebenen Zahlenwert des Volumenanteils der Nanopartikel in dem Komposit im Bereich von 100 bis 50000, stärker bevorzugt 200 bis 30000, noch stärker bevorzugt 300 bis 20000 und am stärksten 500 bis 15000 ist.

In der vorliegenden Erfindung wird die Oberflächenrauigkeit nach ISO4287 ermittelt.

Das erfindungsgemäße Komposit wird aus mindestens zwei Komponenten hergestellt, nämlich aus strukturierbarem Matrixmaterial und Nanopartikeln. Dabei sollen sich die Brechungsindices der Komponenten möglichst wenig unterscheiden. Bevorzugt ist ein Unterschied der Brechungsindices der Komponenten oder ein Unterschied im Brechungsindex des Komposits mit Nanopartikeln und ohne Nanopartikel von weniger als 20%, stärker bevorzugt weniger als 10%, noch stärker bevorzugt weniger als 5% und besonders bevorzugt weniger als 3% bei der Wellenlänge, bei der die Strukturierung durchgeführt wird. Ein anderes Kriterium ist die absolute Differenz der Brechungsindices bei der Wellenlänge, bei der die Strukturierung durchgeführt wird. Diese Differenz sollte weniger als 0,5 sein ist zunehmend bevorzugt weniger als 0,5, weniger als 0,3, weniger als 0,2, weniger als 0,1, weniger als 0,07, weniger als 0,05 oder weniger als 0,02.

Der erfindungsgemäße Kompositansatz enthält ein photostrukturierbares Material und Nanopartikel, wobei der Brechungsindex des photostrukturierbaren Materials sich von dem Brechungsindex der Nanopartikel bei einer Wellenlänge, die aus dem Bereich von 150 nm bis 2000 nm, vorzugsweise 150 nm bis 1000 nm ausgewählt ist, um weniger als 0,5 unterscheidet.

In einer anderen Ausführungsform enthält der Kompositansatz ein photostrukturierbares Material und Nanopartikel, wobei der Brechungsindex des Kompositansatzes mit Nanopartikeln sich von dem Brechungsindex des Kompositansatzes ohne Nanopartikel bei einer Wellenlänge, die aus dem Bereich von 150 nm bis 2000 nm, vorzugsweise 150 nm bis 1000 nm ausgewählt ist, um weniger als 0,5 unterscheidet.

In einer bevorzugten Ausführungsform ist der Kompositansatz so ausgewählt, dass der Brechungsindex des Kompositansatzes mit Nanopartikeln sich von dem Brechungsindex des Kompositansatzes ohne Nanopartikel bei einer Wellenlänge, die aus dem Bereich von 150 nm bis 1000 nm ausgewählt ist, um weniger als 0,5 unterscheidet, und dass in dem nach Photostrukturierung erhaltenen Komposit der Brechungsindex des Komposits mit Nanopartikeln sich von dem Brechungsindex des Komposits ohne Nanopartikel bei einer Wellenlänge, die aus dem Bereich von 150 nm bis 1000 nm ausgewählt ist, um weniger als 0,5 unterscheidet.

Der in der vorliegenden Erfindung genannte Wellenlängenwert von 150 nm bis 2000 nm ist vorzugsweise 150 nm bis 1000 nm und stärker bevorzugt 390 nm bis 1000 nm.

Dieser geringe Wert der Differenz kann allgemein dadurch erzielt werden, dass der Brechungsindex des Kompositansatzes mit Nanopartikeln bei der Wellenlänge, bei der die Strukturierung durchgeführt wird, sich von dem Brechungsindex des Kompositansatzes ohne Nanopartikel um weniger als 0,5 unterscheidet. Dies kann bei einem Unterschied des Brechungsindexes zwischen photostrukturierbarem Material und Nanopartikeln von mehr als 0,5 dadurch erreicht werden, dass der Volumenanteil des photostrukturierbaren Materials entsprechend größer ist als der Volumenanteil der Nanopartikel. Die geeigneten Volumenanteile können durch Vorversuch ermittelt werden. Auf diese Weise können die vorstehend genannten bevorzugten Brechungsindexunterschiede erzielt werden. Wenn es sich beispielsweise bei dem photostrukturierbaren Material um ORMOCER® mit einem Brechungsindex von 1,55 und bei den Nanopartikeln um Magnetit mit einem Brechungsindex von 2,15 handelt, kann das Verhältnis des Volumenanteils des photostrukturierbaren Materials zu dem Volumenanteil der Nanopartikel so groß sein, dass der abweichende Brechungsindex der Nanopartikel wenig Einfluss hat und der gewünschte Brechungsindex eingestellt werden kann. Vorzugsweise ist in dem Beispiel von ORMOCER® und Magnetit dieses Verhältnis 10/1 und stärker bevorzugt 99/1.

Die geringe Differenz der Brechungsindices kann in einem Spezialfall dadurch erzielt werden, dass die Differenz der jeweiligen Brechungsindices der Komponenten, also im Wesentlichen des photostrukturierbaren Materials und der Nanopartikel, bei der Wellenlänge, bei der die Polymerisationsreaktion durchgeführt wird, in den genannten bevorzugten Bereichen liegen. Wenn die Brechungsindices der einzelnen Komponenten sich beispielsweise um weniger als 0,5 unterscheiden, können sie zum Erzielen des gewünschten Brechungsindexes von weniger als 0,5 in dem Kompositansatz in beliebigen Volumenverhältnissen gemischt werden.

Auf diese Weise kann der Fachmann den gewünschten Unterschied im Brechungsindex durch geeignete Auswahl der Komponenten und der Anteile dieser Komponenten in dem Kompositansatz einstellen.

Durch die Strukturierung, also die Polymerisationsreaktion, kann sich insbesondere der Brechungsindex des photostrukturierbaren Materials ändern. Die Beeinflussung der Brechzahl erfolgt dabei über die Verdichtung des Materials durch lichtinduzierte Polymerisationsreaktion und auch über die lichtinduzierte Bildung von chemischen Funktionalitäten mit anderer Polarisierbarkeit. Steuerungsgröße ist hierbei wesentlich die Materialzusammensetzung, die über Parameter wie funktionelle Gruppen, sterische Wechselwirkung, Strukturbildung und dergleichen auf Eigenschaften wie Brechzahl Einfluss nimmt. Zum anderen spielen Initiatorart und -menge sowie die verwendete Energiedosis eine erhebliche Rolle.

Durch die Polymerisationsreaktion kann sich die Differenz der Brechungsindices der Komponenten in dem strukturierten Material von der Differenz der Brechungsindices der Komponenten in dem Kompositansatz aus strukturierbarem Material und Nanopartikeln unterscheiden. Vorzugsweise sollen die Komponenten so ausgewählt werden, dass dieser Differenzunterschied möglichst gering ist. In dem erfindungsgemäßen Komposit unterscheiden sich die Brechungsindices der Komponenten vorzugsweise um weniger als 0,5, stärker bevorzugt weniger als 0,3, weniger als 0,1 oder weniger als 0,05, noch stärker bevorzugt weniger als 0,03 und besonders bevorzugt weniger als 0,01 bei einer Wellenlänge, die aus dem Bereich von 150 nm bis 2000 nm, vorzugsweise 150 nm bis 1500 nm, noch stärker bevorzugt 150 nm bis 1000 nm ausgewählt ist.

In einer weiteren bevorzugten Ausführungsform der vorliegenden Erfindung ist vorgesehen, dass die Nanopartikel mit der Gerüststruktur verbunden sind. In bevorzugter Ausführung ist die Bindung so ausgeführt, dass ein Wechsel des Mediums, in dem das Komposit enthalten ist, beispielsweise ein Kulturmediumwechsel nicht zum Ablösen der Nanopartikel von der Gerüststruktur führt. In bevorzugter Ausführung ist die Bindung waschstabil, d. h. die Nanopartikel werden auch bei Wechsel des Kulturmediums und gegebenenfalls erfolgenden Waschschritten mit üblichen Waschmedien, wie Puffern, nicht von der Gerüststruktur abgelöst.

Bevorzugt ist vorgesehen, dass die Nanopartikel über elektrostatische Wechselwirkungen mit der Gerüststruktur verbunden sind, insbesondere durch ionische Bindung.

In einer weiteren bevorzugten Ausführungsform der vorliegenden Erfindung ist vorgesehen, dass die Nanopartikel durch UV-Crosslinking mit der Gerüststruktur verbunden sind.

Zur stärkeren und gezielten Bindung der Nanopartikel an die Matrix können die Nanopartikel und/oder die Matrixmaterialien funktionalisiert sein und über Verknüpfungsgruppen verbunden sein. Diese Verknüpfungsgruppen können als Abstandshalter dienen. Wenn diese Abstandshalter unterschiedlich lang sind, kann die effektive Oberfläche des Komposits zusätzlich erhöht werden. In einer Ausführungsform kann der Einbau der Nanopartikel in die Matrix und die Verknüpfung mit Nanopartikeln mittels Funktionalisierung mit ggf. unterschiedlich langen Abstandshaltern kombiniert werden.

In einer weiteren bevorzugten Ausführungsform der vorliegenden Erfindung ist vorgesehen, dass das Zellkultursystem zur Durchführung von Migrationsversuchen, insbesondere Migrationsversuche in vitro, eingesetzt wird.

Ein Beispiel eines Komposits besteht aus einer photostrukturierbaren ORMOCER®-Matrix mit eingebetteten Nanopartikeln, beispielweise Silica-Nanopartikel (Fig. 2).

Durch die Photostrukturierbarkeit kann das Material mittels aller photostrukturierbaren Methoden wie 3D-Druck, UV-Lithographie und MPP strukturiert werden.

Durch die Kombination dieser Verfahren ist es möglich, beliebig dreidimensional geformte feinstrukturierte Gerüste, z. B. Scaffolds, im Zentimeterbereich mit einer Strukturierungsauflösung im submikrometer Bereich herzustellen (Fig. 1).

Die gewünschte Struktur kann beispielsweise eine extrazelluläre Matrix eines biologischen Gewebes sein, sodass biologische Zellen in die nachgebildete extrazelluläre Matrix eingebracht werden können.

Durch Zugabe der Nanopartikel ist es möglich, zusätzlich eine intrinsische Oberflächenrauigkeit im Nanometerbereich ohne weiteren Prozessschritt zu realisieren. Diese kann durch den Durchmesser der Nanopartikel beliebig variiert werden. Beispielsweise kann sie an jeden Zelltyp bzw. das gewünschte Zellverhalten angepasst werden.

Die Oberfläche der Nanopartikel kann chemisch funktionalisiert werden, um den Zellen eine geeignete chemische Grenzfläche zu schaffen oder auch um die Nanopartikel kovalent über entsprechende photovernetzbare Gruppen an das Matrixmaterial zu binden. Die Oberfläche der Nanopartikel kann auch mit bioaktiven Materialien bzw. Molekülen funktionalisiert werden wie beispielsweise Kollagen, Fibronektin, Hyaluronsäure, Proteine, DNA, RNA, Antikörper, Integrine, Zellrezeptoren etc. Die Oberfläche oder auch das Volumen der Nanopartikel können mit einem Farbstoff versehen sein um diese als Marker oder im freigesetzten Zustand als Sensor zu nutzen.

Die Matrix kann ebenfalls intrinsisch chemisch funktionalisiert werden.

3-(Trimethoxysilyl)propylmethacrylat ist ein Beispiel eines Silans, das Bestandteil in ORMOCER®en sein kann und als Oberflächenfunktionalisierung von Nanopartikeln dienen kann. Über solche photovernetzbare Gruppen können die Nanopartikel kovalent an das Matrixmaterial gebunden werden.

Durch die Verwendung von Silbernanopartikel wäre es zusätzlich möglich, antimikrobielle Oberflächen auf den 3D-Strukturen zu generieren.

### Brechungsindex (Brechzahl) verschiedener Materialien

ORMOCER®e können in der vorliegenden Erfindung als strukturierbare Matrixmaterialien eingesetzt werden. Es handelt sich dabei um farblose Materialien, die im sichtbaren Bereich keine Absorption zeigen. Der Brechungsindex hängt insbesondere von in den ORMOCER®en eingesetzten Heteroelementen (Ti, Zr) ab. Eine Erhöhung von beispielsweise der Anzahl von Zr-O- oder Ti-O-enthaltenden Strukturen in Epoxysilan-basierten ORMOCER®en erhöht auch den Brechungsindex von 1.48 auf bis zu 1.68. Es sind Materialien auf der Grundlage von Multiacrylatalkoxysilanen mit verschiedenen Abstandshaltergruppen ohne Heteroelemente entwickelt worden, um den Brechungsindex von 1,52 mit linearen aliphatischen auf 1,56 mit aromatischen oder 1,60 mit halogenierten aromatischen Abstandshaltergruppen zu erhöhen. Um den Brechungsindex von ORMOCER®-Systemen auf etwa 1,45 zu senken, können spezielle multifunktionelle Acrylatsysteme oder fluorinierte Silane eingesetzt werden.

Der Brechungsindex eines ORMOCER® Gemisches kann sehr gezielt eingestellt werden indem man ORMOCER®e mit unterschiedlichen Brechungsindizes in geeignetem Verhältnis vermengt. Dies ist in Fig. 6 für die ORMOCER® I und ORMOCER®e II gezeigt.

Beispiele für Vorläufermoleküle von Varianten von ORMOCER®en sind in der folgenden Tabelle 1 zusammengefasst. Diese Varianten sind in Houbertz R. et al. (2003) "Inorganicorganic hybrid materials for application in optical devices", Thin Solid Films 442, 194-200, in Buestrich R. et al. (2001) "ORMOCER®s for Optical Interconnection Technology" Journal of Sol-Gel Science and Technology 20, 181-186 und in EP 1 196 478 B1 beschrieben.

**Tabelle 1**

| Variante von ORMOCER® | Vorläufer |
|---|---|
| ORMOCER® I | Diphenylsilandiol, 3-(Trimethoxysilyl)propylmethacrylat (MEMO) |
| ORMOCER® II | Diphenylsilandiol, 3-(Trimethoxysilyl)propylmethacrylat (MEMO), Trimethoxy(3,3,3-trifluorpropyl)silan |

Die ORMOCER®e weisen die in Tabelle 2 zusammengefassten optischen Eigenschaften auf.

**Tabelle 2**

| | Brechungsindex | |
|---|---|---|
| Wellenlänge | ORMOCER® I | ORMOCER® II |
| 635 nm | 1,5527 | 1,5343 |
| 800 nm | 1,543 | 1,532 |
| 1310 nm | 1,539 | 1,524 |
| 1550 nm | 1,537 | 1,521 |

Diese Werte sind höher als der Brechungsindex für Quarzglas (n = 1,4585 bei 587,6 nm) oder PMMA (n = 1,492 bei 589,3 nm), jedoch niedriger als der Brechungsindex von Polycarbonat (PC) (n = 1,590 bei 589,3 nm).

Silica hat einen Brechungsindex von 1,45. Magnetit hat einen Brechungsindex von 2,4. Partikel von SiO₂ einer Größe von 260 nm bis 680 nm mit einem Gehalt von 0 bis 6,4 Gew.-% Fe₃O₄ weisen einen Brechungsindex von 1,31 bis 1,39 auf.

Anorganische Materialien weisen eine Bandbreite an Brechungsindices auf, z. B. Titandioxid (Rutil) 3,10 (bei 589 nm) und Titanidoxid (Anatas) 2,52 (bei 589 nm), Glas 1,45 bis 2,14 (bei 589 nm), Calciumphosphat 1,63, Calciumhydrogenphosphat 1,35.

Organische Polymere haben Brechungsindices bei 589 nm von 1,585 (Polycarbonat), 1,55 bis 1,63 (Epoxidharz), 1,533 (Cycloolefin-Copolymere), 1,534 (Polymethacrylmethylimid).

### Unterschied im Brechungsindex

In dem erfindungsgemäßen Komposit unterscheidet sich der Brechungsindex des Komposits mit Nanopartikeln von dem Brechungsindex des Komposits ohne Nanopartikel bei der Wellenlänge, bei der die Polymerisationsreaktion durchgeführt wird, um weniger als 0,5 oder einen genannten bevorzugten Wert.

Entsprechendes gilt in dem erfindungsgemäßen Verfahren für den Kompositansatz.

In einer Ausführungsform des Verfahrens unterscheidet sich der Brechungsindex des photostrukturierbaren Materials von dem Brechungsindex der Nanopartikel bei der Wellenlänge der in Stufe (ii) durchgeführten Bestrahlung um weniger als 0,5 oder einen genannten bevorzugten Wert.

Dabei kann das photostrukturierbare Material ein einziges Material oder ein Gemisch von Materialien sein, welches einen gewissen Brechungsindex aufweist.

Entsprechend kann ein Ausdruck wie "Nanopartikel mit einem Brechungsindex von x" eine einzige Spezies von Nanopartikeln oder auch ein Gemisch von Nanopartikeln mit unterschiedlicher Zusammensetzung, unterschiedlicher Größe, unterschiedlichem Brechungsindex und dergleichen bedeuten, wobei das Gemisch den Brechungsindex x aufweist.

Der Unterschied zwischen dem Brechungsindex des Komposits mit Nanopartikeln und dem Brechungsindex des Komposits ohne Nanopartikel in dem erfindungsgemäßen Komposit kann auf verschiedene Weise bestimmt werden.

Wenn die Nanopartikel aus dem Komposit abgetrennt werden können, z. B. durch Auswaschen, kann der Brechungsindex vor und nach dem Abtrennen gemessen werden.

Wenn die Nanopartikel nicht zerstörungsfrei aus dem Komposit abgetrennt werden können, ist es möglich, die Zusammensetzung und die Struktur der Nanopartikel spektrometrisch oder in Kombination mit anderen Analysemethoden zu bestimmen und mit den Analysedaten die gewünschten Nanopartikel herzustellen. Der gemessene Brechungsindex dieser Nanopartikel kann dann von dem Brechungsindex des Komposits abgezogen werden. Der ermittelte Wert gilt in der vorliegenden Erfindung dann als der Unterschied zwischen dem Brechungsindex des Komposits mit Nanopartikeln und dem Brechungsindex des Komposits ohne Nanopartikel.

Entsprechend wäre es möglich, die Struktur und Zusammensetzung der Matrix zu bestimmen, die Matrix herzustellen und deren Brechungsindex zu bestimmen. Aus den gewonnenen Messdaten könnte dann ebenfalls die Brechungsindexdifferenz des Komposits mit Nanopartikeln und ohne Nanopartikel ermittelt werden.

### Herstellung von Matrixstrukturen

Eine Möglichkeit zur Herstellung feinstrukturierter Gerüste bzw. hochkomplexer Strukturen bietet die Stereolithographie. Bei dieser Technik wird eine chemische Reaktion, nämlich Photopolymerisation, durch elektromagnetische Strahlung initiiert. Das zu strukturierende Material beziehungsweise die zu strukturierende Materialformulierung muss folglich die Anforderung erfüllen, mit Licht reagieren zu können. Dadurch findet in den exponierten Bereichen ein Phasenübergang von flüssig zu fest statt. Im nachfolgenden Entwicklungsschritt wird somit nur das verbliebene flüssige Material weggelöst. Mit Hilfe eines Laserstrahls können so schichtweise Strukturen erzeugt werden. Vorteile dieser Methode sind, dass hochorganisierte dreidimensionale Scaffolds mit definierter Porosität, Porengröße und Interkonnektivität der Poren mit hoher Reproduzierbarkeit hergestellt werden können und dies mit nur wenigen Prozessschritten möglich ist.

Eine gute Auflösung bietet ein Spezialfall der Stereolithographie, nämlich die Zwei-Photonen-Polymerisation (two photon polymerization, 2PP). Mit dieser Technik können ultrafeine Strukturen bei hoher Auflösung erzeugt werden. Sie beruht auf der simultanen Absorption von zwei Photonen (two photon absorption, TPA), die den Zerfall der Initiatormoleküle und somit auch die nachfolgende chemische Reaktion zwischen den erzeugten Radikalen und den Monomeren auslöst. Für die Anregung sind aufgrund der simultanen Absorption von zwei Photonen sehr hohe Lichtintensitäten nötig, die durch ultrakurze Laserpulse realisiert werden können. Die Rate der Zwei-Photonen-Absorption hängt dabei nichtlinear von der Intensität ab.

Infolgedessen findet die Polymerisation nur in einem räumlich eng begrenzten Bereich um den Laserfokus herum innerhalb der zu verfestigenden Flüssigkeit statt, wodurch auch Auflösungen von < 100 nm erreicht werden können. Wird der Fokus durch das Material bewegt, werden in den belichteten Bereichen in einem Prozessschritt dreidimensionale Mikrostrukturen erzeugt. Damit lässt sich mit relativ guter Genauigkeit ein nur kleiner Raum innerhalb der zu verfestigenden Flüssigkeit ansteuern, der durch die eingetragene Energie verfestigt wird. Die Herstellung des Formkörpers kann daher innerhalb einer entsprechenden Flüssigkeit erfolgen, nicht mehr (nur) an deren Oberfläche.

In einer bevorzugten Ausgestaltung der Erfindung wird die Flüssigkeit mittels Bestrahlung mit Femtosekunden-Laserpulsen verfestigt. Dabei können übliche polymerisierbare Materialien ultrakurzen Laserpulsen mit hoher Spitzenenergie ausgesetzt werden, z. B. Ti:Saphir-Femtosekundenlaser-Pulsen. Diese werden mit Wellenlängen im Bereich von etwa 800 nm in die zu verfestigenden Harze eingestrahlt.

Als hierfür geeignete Laser lassen sich vorzugsweise Ti-Saphir-Laser einsetzen (entweder mit der fundamentalen Wellenlänge von 780 nm oder, je nach Absorptionsverhalten der zu erhärtenden Flüssigkeit, mit der zweiten Harmonischen bei 390 nm); auch andere NIR-Laser (z.B. mit emittierten Wellenlängen von 800 nm bis etwa 1500 nm) sind geeignet. Aber auch andere Laserbestrahlung ist möglich, sofern die eingesetzte Lichtquelle mit einer Intensität in die Flüssigkeit einstrahlen kann, die für eine Mehrphotonenanregung geeignet ist. Diese Eigenschaft bieten bei moderaten mittleren Leistungen insbesondere Kurzpulslaser. Das auszuhärtende Material muss transparent für die verwendete Laserwellenlänge sein. Wenn das zu verfestigende Material beispielsweise bei 390 nm einer Einphotonen-Polymerisation zugänglich wäre, kann jede Wellenlänge von 400 nm oder darüber für die Zwei- oder Mehrphotonenpolymerisation eingesetzt werden; je nach Harz sind wegen der Transparenzbedingungen vor allem 500-1000 nm geeignet. Bei Verwendung größerer Wellenlängen kann die Polymerisation auch durch eine n-Photonenabsorption initialisiert werden, wobei n größer 2 gilt.

Insbesondere bei Einsatz von Femtosekundenlasern als Strahlungsquelle erhält man Körper/Schichten mit hoher lithographischer Auflösung. Art und Dauer der Bestrahlung liefern darüber hinaus die Möglichkeit, den Vernetzungsgrad gezielt zu variieren, so dass mit ein und demselben Material bei Bedarf unterschiedliche physikalische Eigenschaften (z.B. unterschiedlich schnelle Abbaubarkeit) erzielt werden können. Die Variation des Vernetzungsgrades kann auch innerhalb einer Gerüststruktur erfolgen. Dies kann entweder an bestimmten Stellen ausgeführt sein oder als Gradient vorliegen. Mit diesem Verfahren können nicht nur dreidimensionale Strukturen auf Substraten (Trägermaterialien) gefertigt werden, sondern es können dreidimensionale freitragende Körper vollständig aus dem Volumen heraus gefertigt werden.

Durch das erfindungsgemäße Verfahren ist es möglich, dreidimensionale Formkörper mit einem durchgängigen porösen Netzwerk innerhalb des Formkörpers großflächig durch Lichtinduzierte Vernetzungsprozesse, insbesondere durch die Mehr-Photonen-Absorptions-Technologie über einen weiten Wellenlängenbereich unter Verwendung verschiedenster Laser- und optischer Systeme in-situ herzustellen. Insbesondere lassen sich mit dem beschriebenen Verfahren große Strukturen und Formkörper mit einer Größe bis in den cm-Bereich herstellen.

Durch die Kombination verschiedener photostrukturierender Verfahren können gewünschte hierarchische Strukturen aufgebaut werden. Auf diese Weise ist eine hierarchische Struktur vom Zentimeter- bis zum Nanometerbereich beliebig anpassbar. Beispielsweise können Überstrukturen im Zentimeterbereich durch ein Rapid-prototyping-Verfahren und Unterstrukturen durch Mehr-Photonen-Absorption (MPA) hergestellt werden.

Die Porenstrukturen werden nach Wunsch ausgewählt, z. B. in Abhängigkeit von den Zelltypen, die auf die Trägermatrix aufzubringen sind.

Im Gegensatz zu den stereolithographischen Verfahren, welche einen Gegenstand schichtweise in einem Bad einer durch Strahlungseinwirkung verfestigbaren Flüssigkeit unter Bewegung des Objektes weiter in das Bad hinein verfestigen, wird bei der Erzeugung mittels Femtosekundenlaser-Bestrahlung ein zu erzeugender dreidimensionaler Körper also in nur einem Arbeitsschritt auf einer Oberfläche oder im Volumen produziert.

Die Zwei- oder Mehrphotonenpolymerisation des organischen, über Zwei-Photonen- oder Mehrphotonen-Polymerisation polymerisierbaren Rests kann über eine bzw. mehrere Gruppen erfolgen, die sich radikalisch polymerisieren lassen. Als radikalisch polymerisierbare Gruppen eignen sich nichtaromatische C=C-Doppelbindungen wie Allyl- oder Vinylgruppen, besonders bevorzugt sind allerdings einer Michael-Addition zugängliche Doppelbindungen.

Beispiele von photostrukturierbaren Matrixmaterialien sind anorganisch kondensierbare Silane und deren Kondensate und/oder Polymerisate. Diese Silane enthalten eine(n) oder mehrere über Sauerstoff an das Silicium gebundene Substituenten bzw. Gruppen mit Ester-, ggf. darüber hinaus mit Ether- und/oder Thioetherbindungen, sowie organisch polymerisierbare Einheiten wie C=C-Doppelbindungen oder ringöffnende Systeme, z.B. Epoxygruppen. Die Silane lassen sich anorganisch kondensieren und/oder organisch über die C=C-Doppelbindungen polymerisieren. Eine solche organische Polymerisation kann beispielsweise mit Hilfe von 2- Photonen-Polymerisation (2PP) erfolgen, so dass sich strukturierte, im Wesentlichen monomerfreie Materialien erhalten lassen. Durch Variation des Anteils an anorganisch vernetzbaren Einheiten und/oder an organisch polymerisierbaren Einheiten lassen sich die mechanischen Eigenschaften der aus den Silanen erzeugten Hybridpolymere gezielt so einstellen, dass sie denjenigen natürlicher, weicher oder harter Gewebe ähneln. Diese Hybridpolymere sind besonders geeignet zur Herstellung von Scaffolds.

Die bekannten ORMOCER®-Hybridmaterialien lassen sich sowohl über eine organische Polymerisation von C=C-Doppelbindungen als auch über anorganische Vernetzungsreaktionen (Si-O-Si-Brücken-Bildung) verfestigen, wobei das Vorhandensein von organisch polymerisierbaren C=C-Doppelbindung eine räumliche Strukturierung bei der Polymerisation ermöglicht.

In einer Ausführungsform der Erfindung wird eine gewünschte dreidimensionale Matrixstruktur hergestellt.

Dabei werden in einem ersten Schritt des Verfahrens zur Herstellung einer gewünschten Struktur Daten bereitgestellt, welche den gewünschten strukturellen Aufbau beschreiben. Durch die Daten wird die geometrische Form der Struktur beschrieben.

In einem weiteren Schritt des Verfahrens wird ein Vorläufer eines Biopolymers bereitgestellt. Bei dem Vorläufer handelt es sich um einen Ausgangsstoff im Sinne eines Präkursors des Biopolymers, beispielsweise in Form eines Monomers. Bei dem Biopolymer handelt es sich beispielsweise um ein biokompatibles und/oder bioabbaubares Polymer.

Es erfolgt ein örtlich gezieltes Bestrahlen des Vorläufers mit einer elektromagnetischen Strahlung, wobei das Bestrahlen, insbesondere die Auswahl der zu bestrahlenden Bereiche, gemäß dem durch die Daten beschriebenen strukturellen Aufbau erfolgt. Es werden also genau diejenigen zusammenhängenden Teilmengen der Ebene bzw. des Raumes bestrahlt, welche durch die Daten definiert sind. Die elektromagnetische Strahlung kann beispielsweise durch ein fokussiertes Licht oder durch einen Laserstrahl gebildet sein.

Erfindungsgemäß wird die elektromagnetische Strahlung so bemessen, dass in den bestrahlten Bereichen des Vorläufers eine Zwei- oder Multiphotonen-Absorption stattfindet, durch welche der Vorläufer in den bestrahlten Bereichen zu dem Biopolymer polymerisiert wird, sodass er dort zumindest teilweise verfestigt. Da das Bestrahlen des Vorläufers örtlich gezielt entsprechend dem durch die Daten beschriebenen strukturellen Aufbau erfolgt, wird das gebildete Polymer mit der gewünschten Struktur ausgebildet.

In einer weiteren Ausführungsform des Verfahrens werden Anteile des Vorläufers, welche nach dem Bestrahlen nicht polymerisiert sind, ausgeschwemmt. Durch das Ausschwemmen werden u. a. Hohlräume entleert.

### Nanopartikel

Im Rahmen der vorliegenden Erfindung werden unter Nanopartikeln Partikel verstanden, die einen Durchmesser von 1 bis 10000 nm, vorzugsweise 1 bis 1000 nm aufweisen. Derartige Nanopartikel können aus verschiedenen Materialien aufgebaut sein, beispielsweise anorganischen oder organischen Substanzen. In bevorzugter Ausführungsform können deren Oberflächen chemisch reaktive funktionelle Gruppen umfassen, die mit komplementären funktionellen Gruppen von zu bindenden Wirkstoffen affine Bindungen, also kovalente und/oder nicht-kovalente Bindungen, eingehen und auf diese Weise die Wirkstoffe an ihren Oberflächen stabil fixieren können. Die vorliegende Erfindung sieht in einer weiteren bevorzugten Ausführungsform vor, dass die Nanopartikel auch Bindungen mit der Gerüststruktur eingehen können. Derartige Bindungen sind bevorzugt elektrostatische Wechselwirkungen oder kovalente Bindungen.

In einer bevorzugten Ausführungsform weisen die Nanopartikel einen mittleren Durchmesser von 1 bis 10000, vorzugsweise von 5 bis 1000 nm, vorzugsweise von 20 nm bis 900 nm, bevorzugt von 30 bis 600 nm, auf. In einer weiteren bevorzugten Ausführungsform weisen die Nanopartikel einen mittleren Durchmesser von 30 bis 500 nm auf.

Der in der vorliegenden Erfindung verwendete Begriff "mittlere Größe" oder "mittlerer Durchmesser" von Nanopartikeln ist der auf die Anzahl der Teilchen bezogene Median des Teilchendurchmessers D_{n,50}.

In einer weiteren Ausführungsform ist es vorgesehen, dass die Nanopartikel aus anorganischen Substanzen, zum Beispiel Gold oder anderen Edelmetallen oder Metallen oder Metalloxiden, Calciumphosphat und Calciumhydrogenphosphat oder anderen Mischphosphaten, Siliziumbasierten oxidischen Materialien, wie Silicaten, Siliziumoxiden, wie Siliziumdioxid, aufgebaut sind. In bevorzugter Ausführung können die Nanopartikel auch DynaBeads sein.

In einer Ausführungsform sind die Nanopartikel mesoporöse Silicananopartikel. In einer weiteren Ausführungsform handelt es sich um Januspartikel.

In einer bevorzugten Ausführungsform sind die Nanopartikel im Volumen oder an der Oberfläche mit einem Fluoreszenzfarbstoff versehen. Dadurch kann die Gerüststruktur durch ein Fluoreszenzmikroskop abgebildet werden. Im Fall einer bioabbaubaren Matrix können die freigesetzten Nanopartikel als Marker bzw. Sensor dienen. Beispielsweise kann der Grad des Gerüstabbaus fluoreszenzmikroskopisch einfach erfasst werden. Werden die Nanopartikel von Zellen aufgenommen können sie als zellinterner Sensor verwendet werden. Werden die Farbstoffe zusätzlich an Antikörper gekoppelt lassen sich die Nanopartikel erst nach erfolgreicher Antigenbindung über das Fluoreszenzsignal erfassen und so zahlreiche biologische Vorgänge untersuchen bzw. erfassen.

In einer weiteren Ausführungsform bestehen die Nanopartikel aus magnetischen, piezoelektrischen oder anderen magnetisch-/elektroaktiven Materialien wie Eisen, Magnetit, Bariumtitanat oder Polyvinylidenfluorid um Strukturen herzustellen die durch magnetische oder elektrische Felder aktuierbar sind.

In einer bevorzugten Ausführungsform ist vorgesehen, dass die Nanopartikel aus organischen Materialien, insbesondere organischen Polymeren, aufgebaut sind. Die Nanopartikel können bevorzugt im Wege der Emulsionspolymerisation hergestellt werden.

Bevorzugt werden Nanopartikel in dem erfindungsgemäßen Komposit eingesetzt, die aus bioabbaubaren Polymeren aufgebaut sind. Weiterhin bevorzugt ist auch eine Verwendung von Nanopartikeln, die einen Durchmesser von 50 nm und eine bioabbaubare Matrix aufweisen.

In einer weiteren bevorzugten Ausführungsform ist es vorgesehen, dass die Nanopartikel aus Polylactiden (PLA), Poly(lactid-co-glycolid)en (PLGA), Polycaprolactonen (PCL), Polyglycoliden, Di- und Tri-Block-Polymeren bspw. PCL/PGA-Di-Block-Systemen, Polyorthoestern (POE), Polyanhydride, Polyhydroxyalkanoaten (PHA), Polypyrrolen (PPy), Polypropylencarbonat, Polyethylencarbonat, Polyalkylcyanonitril oder Polyethylenglycol aufgebaut sind.

Bevorzugt ist vorgesehen, dass die Nanopartikel je nach gewünschtem Freisetzungsprofil des Wirkstoffs Polymere mit unterschiedlichem Molekulargewicht und variabler Polarität aufweisen. Die Auswahl des für den Aufbau des Nanopartikels einzusetzenden Materials kann in bevorzugter Ausführungsform danach erfolgen, in welcher Form und Kinetik der Wirkstoff freigesetzt werden soll.

In einer weiteren bevorzugten Ausführungsform der vorliegenden Erfindung ist es vorgesehen, dass die Nanopartikel durch Kopplung mit funktionellen Gruppen funktionalisiert worden sind. In besonders bevorzugter Ausführungsform ist vorgesehen, dass die Nanopartikel selbst funktionelle Gruppen auf ihrer Oberfläche aufweisen.

Der in der vorliegenden Erfindung verwendete Ausdruck "Nanopartikel" bedeutet eine einzige Art von Nanopartikeln oder ein Gemisch verschiedener Nanopartikel. Der Unterschied der Nanopartikel in dem Gemisch ist mindestens einer, der aus der Gruppe ausgewählt ist, die aus der Zusammensetzung der Nanopartikel, der Größe der Nanopartikel und dem Brechungsindex der Nanopartikel besteht. Beispielsweise heißt das, dass ein Ausdruck wie "Nanopartikel mit einem Brechungsindex von x" auch ein Gemisch von Nanopartikeln mit unterschiedlicher Zusammensetzung, unterschiedlicher Größe, unterschiedlichem Brechungsindex und dergleichen bedeuten kann, wobei das Gemisch den Brechungsindex x aufweist.

### Beispiele

Die vorliegende Erfindung wird anhand der nachfolgenden Beispiele weiter veranschaulicht.

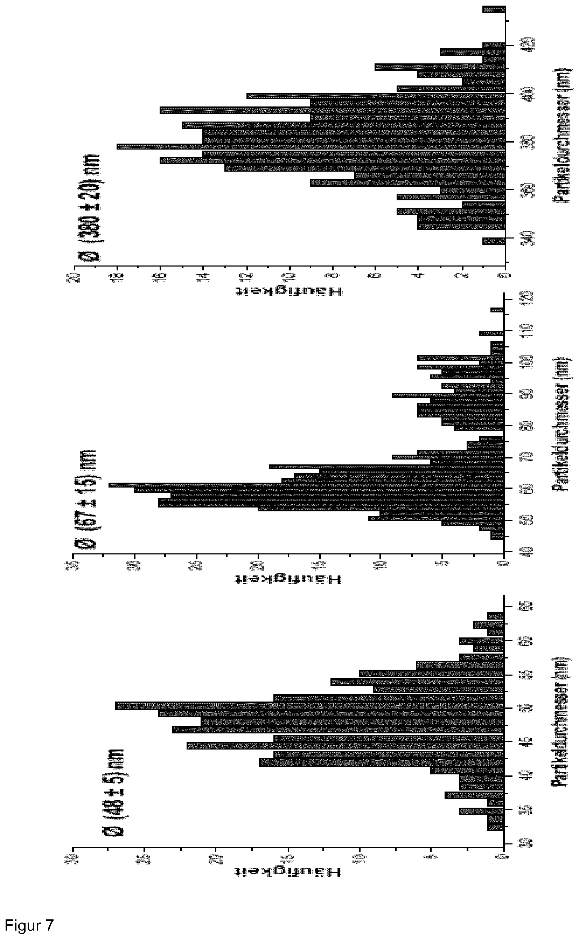

Für das folgende Ausführungsbeispiel wurde als Matrixmaterial das vorstehend beschriebene ORMOCER® I aus der ORMOCER® Familie verwendet, dass eine sehr gute Biokompatibilität zeigt. Zur Herstellung von ORMOCER® I wurde zusätzlich zu der in Buestrich et al. (2001) offenbarten Formulierung 2 Gew.-% Irgacure 369 als Photostarter zugegeben (vgl Houbertz et al. (2003). Als Füllstoff wurden Silica-Nanopartikel (SNP) verwendet, die mittels Stöberprozess hergestellt wurden. Die Partikel wurden in die Matrix eingerührt und im Ultraschallbad homogenisiert. Um den Einfluss der Oberflächenrauigkeiten auf das Zellverhalten zu zeigen, wurden Kompositansätze aus ORMOCER® I und SNP mit einem Füllgrad von bis zu 20 Vol.-% nach oben beschriebenen Verfahren hergestellt (Tab. 3). Dabei wurde der Durchmesser der SNP variiert und die Partikelgrößenverteilung mittels Rasterelektronenmikroskopie bestimmt (Figur 7).

Die Ergebnisse sind in der folgenden Tabelle 3 zusammengefasst.

Tabelle 3: Zusammensetzung der hergestellten ORMOCER® I-SNP Komposite sowie die Ergebnisse der mittleren Partikeldurchmesser und Oberflächenrauigkeiten der gerakelten Schichten. Der angegebene Fehler ergibt sich aus der Standardabweichung der Messungen.

| Ansatz | OC-A | OC-B | OC-C | OC-D | OC-E |
|---|---|---|---|---|---|
| Matrixmaterial | ORMOCER® I | ORMOCER® I | ORMOCER® I | ORMOCER® I | ORMOCER® I |

| Silica-Nanopartikel | | | | | |
|---|---|---|---|---|---|
| Füllgrad [Vol.-%] | 0 | 20 | 20 | 20 | 10 |
| D_{n,50} (nm) | - | 48 ± 5 | 67 ± 15 | 380 ± 20 | 67 ± 15 |

| Gerakelte Schichten | | | | | |
|---|---|---|---|---|---|
| Z_{q} (nm) | 0,8 ± 0,1 | 1,5 ± 0,1 | 4,2 ± 0,1 | 38 ± 1 | |
| Z_{z}(nm) | 6,9 ± 2,3 | 25 ± 12 | 41 ± 1 | 316 ± 45 | |

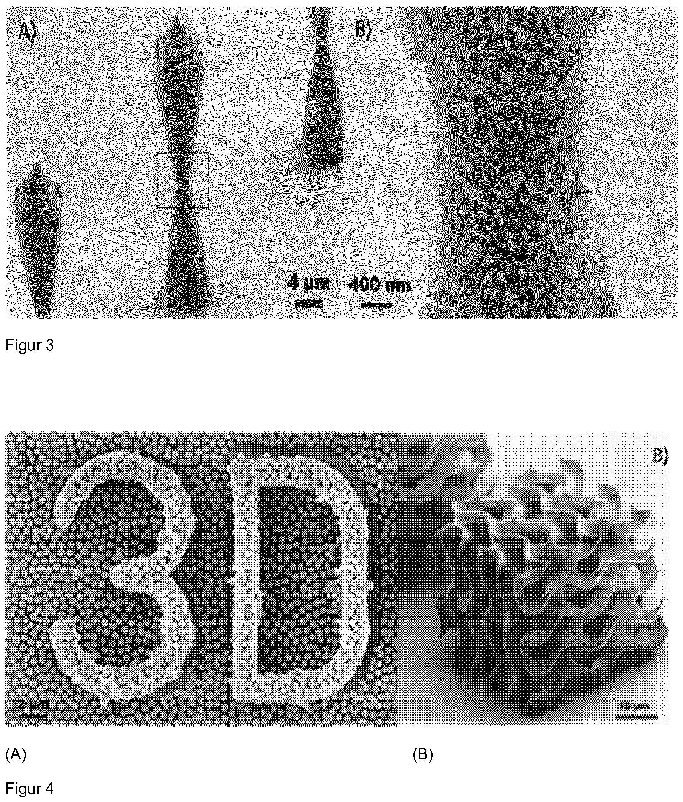

Figur 3 zeigt beispielhaft eine 3D-Struktur aus dem Ansatz OC-E, die mittels 2PP hergestellt wurde. Zur Strukturierung wurde ein gepulster 80 MHz Ti:Sapphire Laser (Coherent Ultra II) bei einer Wellenlänge von 705 nm verwendet. Figur 3B zeigt einen Ausschnitt aus Figur 3A und verdeutlicht die Rauigkeit der Oberfläche an der 3D-Struktur.

Auch aus den Kompositen mit einem Füllgrad von 20 Vol.-% konnten komplexe 3D-Strukturen mit einer hohen Auflösung hergestellt werden. Hierbei kann sowohl das klassische Verfahren, bei dem die Probe lateral und das Objektiv axial mechanisch bewegt werden (Figur 4A), als auch die Scannermethode verwendet werden, bei dem der Fokus über Spiegel mit Galvanometerantrieb lateral abgelenkt wird und nur das Objektiv mechanisch in axialer Richtung bewegt wird (Figur 4B). Für die Gyroidstruktur in Figur 4B wurde der Laser T-Pulse der Marke Amplitude (Wellenlänge nach Frequenzverdopplung: 515 nm) mit einer Scannereinheit des Herstellers Lightfab verwendet.

Figur 5 verdeutlicht den Vorteil der hierarchischen Strukturierung des Komposits, wodurch es möglich ist, verschiedene Photostrukturierungsverfahren innerhalb einer Probe zu vereinen und somit Dimensionen im Zentimeterbereich (3D-Druck), Millimeterbis Mikrometerbereich (Lithographie) und Submikrometerbereich (MPP) zu realisieren. Über die Nanopartikel wird dies bis in den Nanometerbereich ergänzt. Für das vorliegende Ausführungsbeispiel wurden Linien (Länge: 1mm, Breite: 100 µm, Höhe 15 µm) aus ORMOCER® I lithographisch auf einem Deckglas strukturiert. Anschließend wurden die Linien mit OC-D überschichtet und in den Zwischenraum mittels MPP gekreuzte Linien (Linienbreite: 6 µm, Gitterperiode: 15 µm) strukturiert. Zur Strukturierung wurde ein gepulster 80 MHz Ti:Sapphire Laser (Coherent Ultra II) bei einer Wellenlänge von 705 nm verwendet und mit einem Ölimmersionsobjektiv (Nikon, NA=1,45) bei einer Leistung von 6 mW in das Material fokussiert und die Probe mit einer Geschwindigkeit von 1 mm/s bewegt.

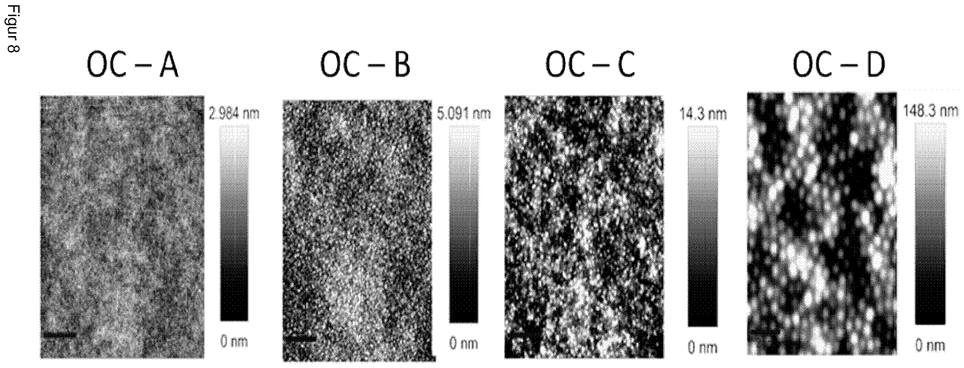

Aus den Kompositen wurden mittels Rakelmethode, unter Reinraumbedingungen, ebene Schichten mit einer Dicke von ca. 10 µm hergestellt und durch UV-Bestrahlung vernetzt. Die Oberflächenrauigkeit wurde mittels Rasterkraftmikroskopie bestimmt und mit der einer ORMOCER® I-Schicht ohne Füllstoff verglichen (Figur 8). Diese zeigt eine Rauigkeit von nur Z_{z} = (6,9 ± 2,3) nm was durch Zugabe der Nanopartikel auf bis zu Z_{Z} = (316 ± 45) nm erhöht werden konnte (Tab. 3).

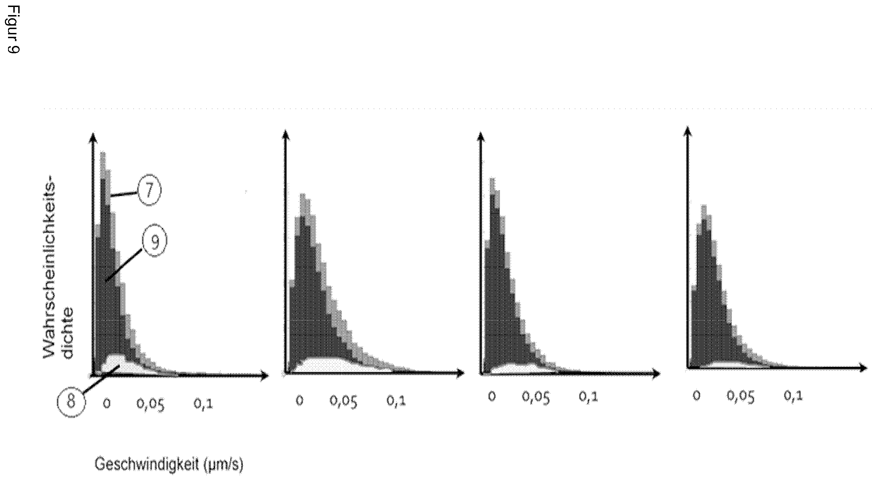

Auf den vorstehend beschriebenen Schichten wurden fluoreszenzmarkierte, freeGFP exprimierende D. Dictyostelium (HG1694) Zellen kultiviert und mittels Time-Lapse Fluoreszenzmikroskopie eine Migrationsanalyse (nach Gorelashvili et. al, Amoeboid migration mode adaption in quasi-3D spatial density gradients of varying lattice geometry, New Journal of Physics, 16, 2014) durchgeführt. Dabei zeigte sich, dass mit zunehmender Oberflächenrauigkeit die aktiven Migrationsphasen abnehmen und die Durchschnittsgeschwindigkeit der Zellmigration zunimmt, somit gezielt Einfluss auf das Migrationsverhalten genommen werden kann (Abb. 9).

In einem weiteren Versuch wurde gezeigt, dass eine qualitativ hochwertige Strukturierierung von Kompositen bei hohem Brechzahlunterschied der Komponenten möglich ist, wenn eine Komponente nur in geringer Konzentration in dem Kompositansatz enthalten ist. Komposite mit hohem Brechzahlunterschied der Komponenten werden beispielsweise hergestellt, wenn durch die Wahl der Nanopartikel weitere Materialeigenschaften in eine Struktur eingebracht werden sollen. Es handelt sich hier beispielsweise um Silbernanopartikel, um antibakterielle Oberflächen zu schaffen, was für Implantate einen großen Vorteil darstellt. Mittels Bariumtitanatpartikel ist es möglich, aktorische Strukturen herzustellen, um mechanische Reize auf Zellen auszuüben. Durch den Einsatz von Magnetitpartikeln lassen sich mittels externer Magnetfelder bewegliche Strukturen erzeugen, oder die Magnetitpartikel können als Kontrastmittel für MRT oder MPI eingesetzt werden, um die Strukturen zu überwachen.

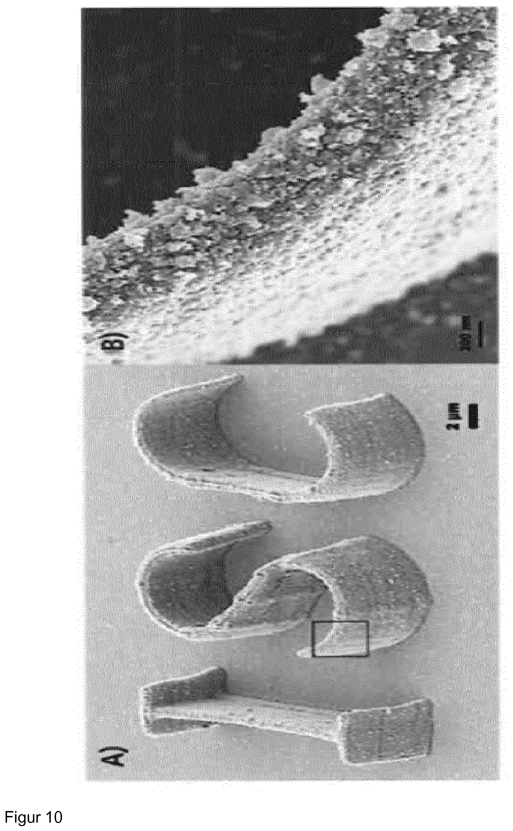

Durch den hohen Brechzahlunterschied von ORMOCER® I (1,55 bei 589 nm) und beispielsweise Magnetit (2,15 bei 589 nm) wird die Qualität des Fokus und damit das Auflösungsvermögen der Strukturierung zwar beeinträchtigt, die Qualität kann aber durch eine Anpassung des Füllgrades, der Laserwellenlänge bzw. der Laserleistung bis zu einem gewissen Grad ausgeglichen werden. Solche Anpassungen sind erforderlich, wenn nicht-transparente und stark absorbierende Materialen strukturiert werden sollen. Im vorliegenden Fall wurde ein Komposit aus ORMOCER® I und superparamagnetischen Magnetitnanopartikel (Partikelgröße 10-15 nm) mit einem Füllgrad von 1 Vol.-% nach der vorstehend beschriebenen Vorgehensweise hergestellt. Zur Strukturierung wurde ein gepulster 80 MHz Ti:Sapphire Laser (Coherent Ultra II) bei einer Wellenlänge von 705 nm verwendet und mit einem Ölimmersionsobjektiv (Nikon, NA=1,45) bei einer Leistung von 3,75 mW in das Material fokussiert und die Probe mit einer Geschwindigkeit von 0.1 mm/s bewegt. Durch die oben erwähnten Anpassungen, insbesondere durch den geringen Füllgrad, konnte auch in diesem Fall eine detailtreue Strukturierung erhalten werden (vgl. Figur 10).

### Zytotoxizität der verwendeten Komposite

Die Komposite aus Tabelle 3, bestehend aus Silica und ORMOCER® I, wurden für Versuche mit Säugetierzellen verwendet und nach ISO 10993 mit einem Extrakttest auf Zytotoxizität geprüft.

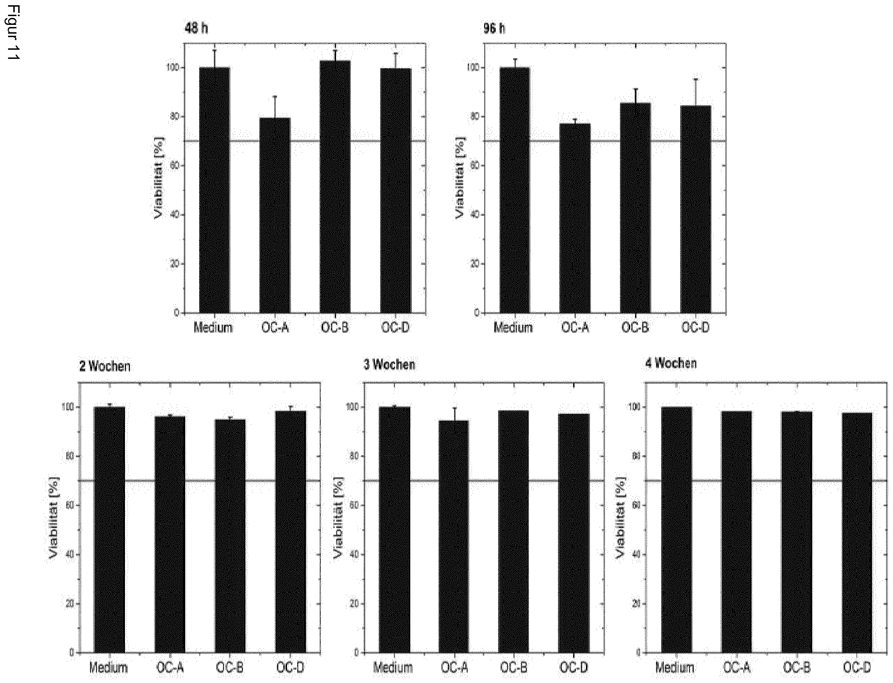

Hierfür wurden Scheiben mit einer Dicke von 120 µm aus OC-A und den Kompositen OC-B und OC-D hergestellt. Diese wurden für verschiedene Zeiträume in DMEM-Medium bei 37 °C, wasserdampfgesättigter und mit 5% CO₂ angereicherter Atmosphäre auf einem Schüttler kultiviert. Anschließend wurden murine L929 Fibroblasten 48 Stunden in dem Extrakt kultiviert und die Viabilität mittels WST-1-Test unter Verwendung von 4-[3-(4-Iodophenyl)-2-(4-nitrophenyl)-2H-5-tetrazolio]-1,3-Benzol-Disulfonat bestimmt. Als Negativkontrolle wurden Zellen in einem DMEM-Medium ohne Kompositproben kultiviert, und deren Zellviabilität ergab den 100%-Referenzwert. Die Ergebnisse für verschiedene Extraktionszeiten sind in Figur 11 gezeigt. Die rote Linie bei einer Viabilität von 70% kennzeichnet die Grenze ab der nach ISO 10993 ein Prüfstoff als nicht zytotoxisch einzuordnen ist. Dieses Kriterium bestehen alle drei getesteten Materialen für alle untersuchten Inkubationszeiten, was ein starkes Indiz für eine hohe Biokompatibilität darstellt.

### Autoklavierbarkeit der mittels TPA hergestellten Kompositstrukturen

Es wurde die Autoklavierbarkeit eines Komposits untersucht, das heißt die Behandlung mit Wasserdampf bei 121 °C bei 1 bar Überdruck für 20 min.

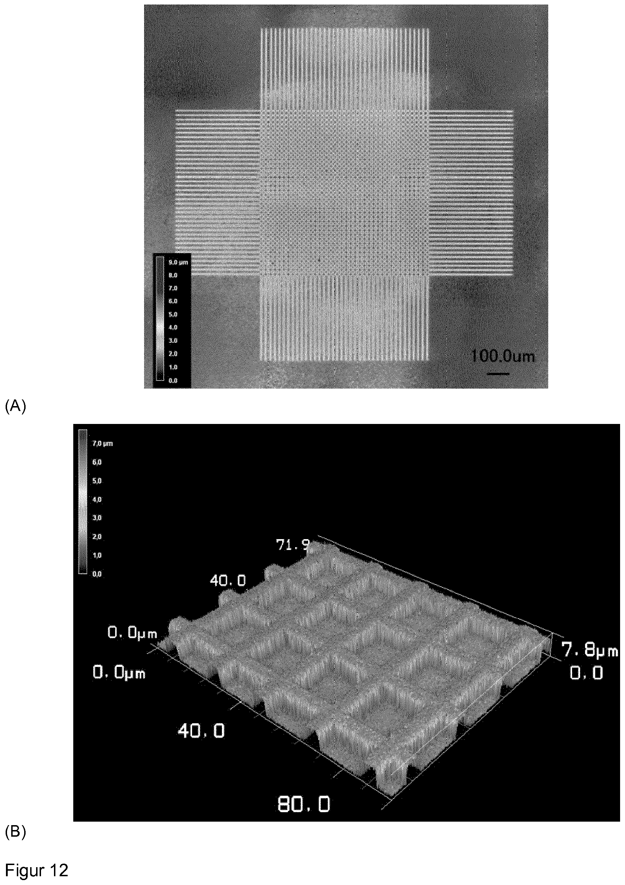

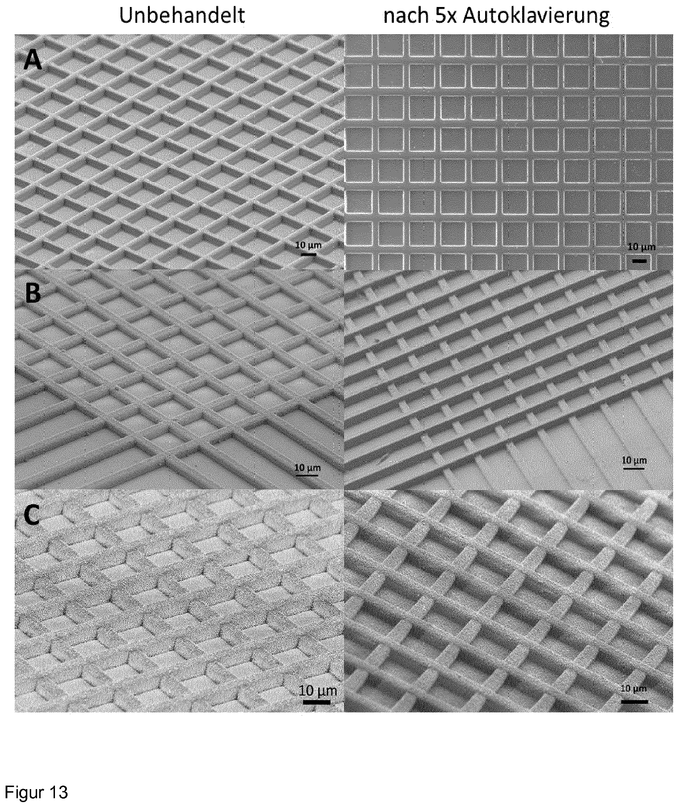

Dazu wurde auf einem Glasträger zunächst eine ca. 10 µm dicke Kompositschicht aufgerakelt, durch UV vernetzt und anschließend darauf mittels TPA eine 1,5 x 1,5 mm Gitterstruktur aus gekreuzten Linien mit einer Höhe und Breite von 5 µm aufgebracht (Figur 12). Diese Proben wurden fünfmal autoklaviert (Systec V-65, Systec GmbH, Linden) und anschließend rasterelektronenmikroskopisch untersucht (Figur 13). Dabei konnten keine strukturellen oder topographischen Veränderungen festgestellt werden.

### Morphologieanalyse von 3T3-Zellen auf mittels TPA hergestellten Kompositstrukturen

Es wurden 3T3-Zellen auf die in Figur 12 gezeigten Strukturen ausgesät und nach 48 Stunden Inkubation mit einem konfokalen Fluoreszenzmikroskop untersucht.

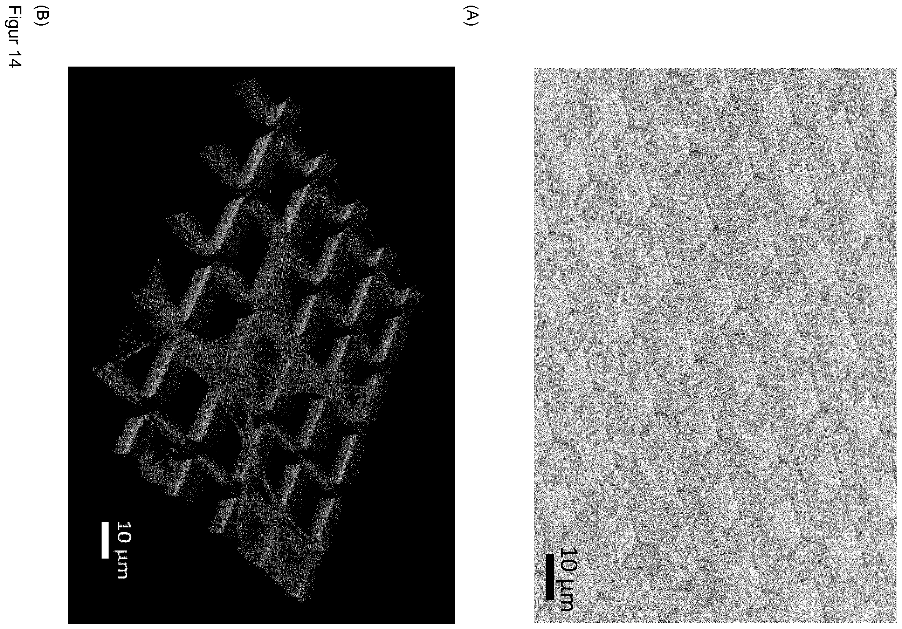

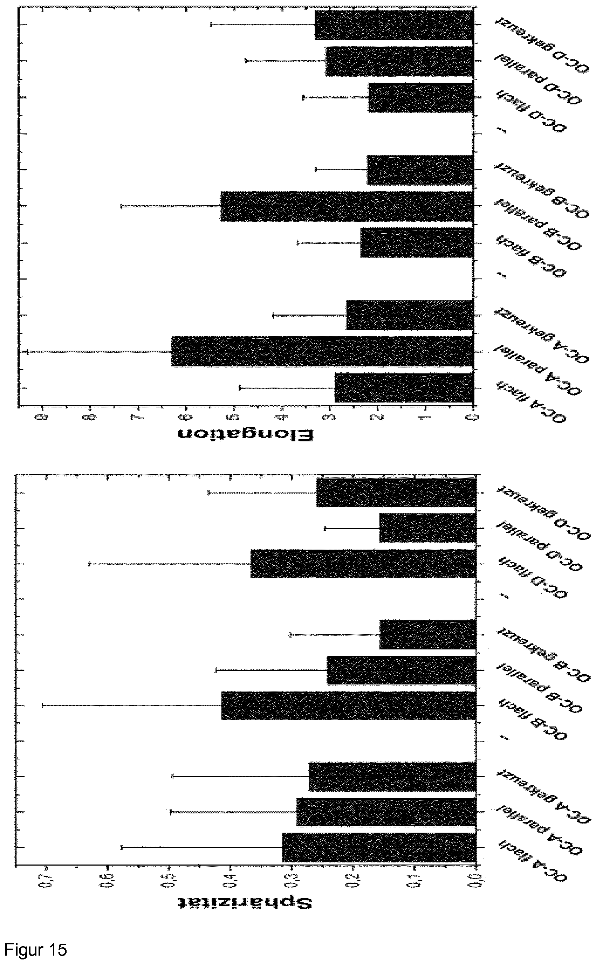

Bei den verwendeten 3T3-Zellen handelte es sich um stabil transfizierte Mausfibroblasten, deren Zytoskelett am Aktin mit dem Farbstoff LifeAct-mCherry markiert ist. Sie wurden in DMEM-Medium (Life Technologies Corporation, USA) kultiviert, das mit 2 mM L-Glutamin (Life Technologies Corporation, USA), 10% fetalem Kälberserum (Bio&SELL GmbH, Feucht/Nürnberg) und 1% Penicillin-Streptomycin angereichert war. Die Proben wurden zunächst in einer 6er-Mikrotiterplatte platziert, mit einer phosphatgepufferten Salzlösung gespült und eine Stunde mit DMEM-Medium kultiviert. Anschließend wurden 2 ml Zellsuspension mit einer Dichte von 1x10⁵ Zellen/ml auf die Strukturen pipettiert. Die Zellen wurden für mindestens 48 Stunden inkubiert und anschließend am Fluoreszenzmikroskop (TCS SP8 X, Leica, Wetzlar) dreidimensional untersucht (Figur 14). Die 3D Aufnahmen wurden mit der Software Fiji (basierend auf ImageJ, National Institutes of Health, USA) mit diversen Filtern bearbeitet und binäre 3D-Bilder erzeugt. Diese wurden mit dem Plugin MorphoLibJ ausgewertet, um die Morphologie der Zellen auf den unterschiedlichen Substraten zu vergleichen. Für jedes Komposit wurden Zellen auf den unstrukturierten Bereichen, den parallelen Linien und den gekreuzten Linien verglichen. In Figur 15 sind die Sphärizität und die Elongation der Zellen in 3D gezeigt. Die Sphärizität der Zellen auf OC-A wurde durch die Linienstrukturen kaum beeinflusst. Demgegenüber zeigt sich für OC-B und OC-D ein starker Einfluss der Partikel. In den flachen Bereichen ist die Sphärizität gegenüber OC-A stark erhöht, während sie in den strukturierten Bereichen verringert ist. Die Elongation ist für OC-A und OC-B besonders für die parallelen Linien sehr hoch, da sich die Zellen zu den Linien ausrichten. Nur in OC-D ist dies nicht der Fall, und der Einfluss der Oberflächenrauigkeit ist scheinbar höher als die Mikrostrukturen der Linien. Insgesamt konnte gezeigt werden, dass durch die Oberflächenrauigkeit als auch durch eine Mikrostrukturierung Einfluss auf das Zellverhalten genommen werden kann. Dies ist hier innerhalb einer Probe ohne weiteren Prozessschritt realisierbar.

### TPA-Kompositstrukturen aus biodegradierbarem ORMOCER® mit farbstoffgefüllten mesoporösen Silicapartikeln

Es wurden mesoporöse Silicananopartikel mit einem Durchmesser von 80 nm mit dem Farbstoff 5(6)-Tetramethylrhodaminisothiocyanat gefüllt und mit einer Hülle aus Polyvinylpyrrolidon (PVP) versehen. Als Matrixmaterial wurde das in WO2016037871 A1 beschriebene, partiell biodegradierbare ORMOCER® verwendet. Die Partikel wurden mit einem Füllgrad von 10 Vol.-% in die Matrix eingerührt und im Ultraschallbad homogenisiert. Die PVP-Hülle schützt den Farbstoff vor dem Austreten, solange die Nanopartikel in der ORMOCER®-Matrix vorliegen. Kommt die Hülle aber später mit dem wässrigen Medium in Kontakt, wird sie aufgelöst und der Wirkstoff kann freigesetzt werden. Zur Strukturierung wurde ein gepulster 80 MHz Ti:Sapphire Laser (Coherent Ultra II) bei einer Wellenlänge von 705 nm verwendet und mit einem Ölimmersionsobjektiv (Nikon, NA=1,45) bei einer Leistung von 5 mW in das Material fokussiert und die Probe mit einer Geschwindigkeit von 0,1 mm/s bewegt. Figur 16 zeigt eine dreidimensionale Fluoreszenzmikroskopabbildung der erhaltenen Struktur sowie eine Rasterelektronenaufnahme der Oberfläche, auf der die Einbindung der Partikel in der Matrix zu sehen ist.

### Bezugszeichenliste

1: (Stereo-)lithographisch, 3D-gedruckte oder mittels anderer Rapid-prototyping-Verfahren hergestellte Überstrukturen
2: MPA gefertigte Submikrometer Strukturen
3: Photostrukturierbare ORMOCER® Matrix
4: (Mesoporöse) Nanopartikel
5: Chemische Oberflächenfunktionalisierung
6: Wirkstoff
7: Gesamtwert der Migrationsphase
8: Aktive Migrationsphasen
9: Passive Migrationsphasen

## Patentansprüche

1. Komposit,
herstellbar durch Photostrukturieren eines photostrukturierbaren Matrixmaterials in einem das photostrukturierbare Matrixmaterial und Nanopartikel in einem Volumenanteil von mindestens 1% enthaltenden Kompositansatz unter Bildung einer strukturierten Matrix mit darin enthaltenen Nanopartikeln, wobei der Brechungsindex des Komposits mit Nanopartikeln sich von dem Brechungsindex des Komposits ohne Nanopartikel bei einer Wellenlänge, die aus dem Bereich von 150 nm bis 2000 nm ausgewählt ist, um weniger als 0,5 unterscheidet,
wobei das Komposit hierarchisch aufgebaut ist und mindestens eine Struktureinheit (I) einer aus dem Bereich von 10 µm bis 100 mm ausgewählten Dicke (i) und von der Struktureinheit (I) abzweigende Struktureinheiten (II) einer jeweils aus dem Bereich von 100 nm bis 1000 µm ausgewählten Dicke (ii) umfasst, wobei an den Abzweigungen die Dicke (ii) höchstens die Hälfte der Dicke (i) ist.

2. Komposit nach Anspruch 1, welches mindestens eine Struktureinheit (I) einer aus dem Bereich von 100 µm bis 50 mm ausgewählten Dicke (i), von der Struktureinheit (I) abzweigende Struktureinheiten (II) einer jeweils aus dem Bereich von 10 µm bis 1000 µm ausgewählten Dicke (ii) sowie von den Struktureinheiten (II) abzweigende Struktureinheiten (III) einer jeweils aus dem Bereich von 100 nm bis 100 µm ausgewählten Dicke (iii) umfasst, wobei an den Abzweigungen der Struktureinheiten (II) von der Struktureinheit (I) die Dicke (ii) höchstens die Hälfte der Dicke (i) ist und an den Abzweigungen der Struktureinheiten (III) von den Struktureinheiten (II) die Dicke (iii) höchstens die Hälfte der Dicke (ii) ist.

3. Komposit nach Anspruch 1 oder 2, worin eine Vielzahl von Struktureinheiten (I), (II) und (III) und eine Vielzahl von Abzweigungen der Struktureinheiten (II) von (I) und (III) von (II) vorhanden ist.

4. Komposit nach einem der vorstehenden Ansprüche, worin Struktureinheiten (I), (II) und (III) jeweils unabhängig voneinander flächig sind.

5. Komposit nach einem der vorstehenden Ansprüche, dessen innere Oberfläche mindestens doppelt so groß wie die äußere Oberfläche ist.

6. Komposit nach einem der vorstehenden Ansprüche, dessen mittels Rasterkraftmikroskopie bestimmte Oberflächenrauigkeit Z_{q} 1 bis 100 nm beträgt.

7. Komposit nach einem der vorstehenden Ansprüche, wobei die mittlere Größe D_{n,50} der Nanopartikel aus dem Bereich von 10 bis 1000 nm ausgewählt ist.

8. Komposit nach einem der vorstehenden Ansprüche, wobei die durch das photostrukturierte Matrixmaterial gebildete Matrix eine Struktur aufweist, deren Größe aus dem Bereich von 0,1 bis 100 µm ausgewählt ist, wobei die Strukturgröße der Matrix und die Größe der Nanopartikel so ausgewählt sind, dass in den Hohlräumen der Matrix jeweils eine Vielzahl von Nanopartikeln enthalten sein kann.

9. Komposit nach einem der vorstehenden Ansprüche, wobei die Nanopartikel in einem Volumenanteil von 10 bis 60% enthalten sind und die mittlere Größe der Nanopartikel aus dem Bereich von 30 bis 600 nm ausgewählt ist.

10. Verfahren zum Herstellen eines Komposits nach einem der Ansprüche 1 bis 9, das strukturiertes Matrixmaterial und darin enthaltene Nanopartikel enthält, welches folgende Stufen umfasst:
(a) eine Stufe, in der ein photostrukturierbares Material und Nanopartikel enthaltender Kompositansatz bereitgestellt wird,
(b) eine Stufe, in der mindestens eine Polymerisationsreaktion durch Bestrahlen des photostrukturierbaren Materials unter Bildung einer Matrix durchgeführt wird, wobei das Komposit erhalten wird,
**dadurch gekennzeichnet, dass** bei der Wellenlänge der Bestrahlung, die in der mindestens einen Polymerisationsreaktion in Stufe (b) durchgeführt wird, der Brechungsindex des Kompositansatzes mit Nanopartikeln sich von dem Brechungsindex des Kompositansatzes ohne Nanopartikel um weniger als 0,5 unterscheidet.

11. Verfahren nach Anspruch 10, wobei Stufe (b) zwei Polymerisationsreaktionen, wenn Struktureinheiten (I) und (II) gebildet werden, oder drei Polymerisationsreaktionen umfasst, wenn Struktureinheiten (I), (II) und (III) gebildet werden, wobei in den jeweiligen Polymerisationsreaktionen jeweils ein Teil der photopolymerisierbaren Materialien umgesetzt wird.

12. Verfahren nach Anspruch 11, wobei die zwei oder drei Polymerisationsreaktionen durch Vorhandensein unterschiedlicher photostrukturierbarer Materialien und/oder durch Variation mindestens eines Verfahrensparameters bewirkt werden, der unter der Bestrahlungswellenlänge, Bestrahlungsleistungsdichte und Bestrahlungsdauer ausgewählt ist.

13. Verfahren nach einem der Ansprüche 10 bis 12, wobei Struktureinheiten (I) durch eine Polymerisationsreaktion und Struktureinheiten (II) durch eine weitere Polymerisationsreaktion sowie, falls vorhanden, Struktureinheiten (III) durch eine weitere Polymerisationsreaktion gebildet werden.

14. Verwendung des Komposits nach einem der Ansprüche 1 bis 9 als Gerüst für biologische Zellen.

## Claims

1. A composite,
obtainable by photostructuring a photostructurable matrix material in a composite batch containing said photostructurable matrix material and nanoparticles in a volume fraction of at least 1% to form a structured matrix having nanoparticles contained therein, wherein the refractive index of said composite having nanoparticles differs by less than 0.5 from the refractive index of said composite having no nanoparticles at a wavelength selected from the range of 150 nm to 2000 nm,
wherein the composite is hierarchically structured and comprises at least one structural unit (I) of a thickness (i) selected from the range of 10 µm to 100 mm and structural units (II) branching off from the structural unit (I) of a thickness (ii) respectively selected from the range of 100 nm to 1000 µm, wherein the thickness (ii) at the branch-off points is at most half the thickness (i).

2. The composite according to claim 1 which comprises at least one structural unit (I) of a thickness (i) selected from the range of 100 µm to 50 mm, structural units (II) branching off from the structural unit (I) of a thickness (ii) respectively selected from the range of 10 µm to 1000 µm, and structural units (III) branching off from the structural units (II) of a thickness (iii) respectively selected from the range of 100 nm to 100 µm, wherein the thickness (ii) of the structural units (II) at the branch-off points from the structural unit (I) is at most half the thickness (i) and the thickness (iii) of the structural units (III) at the branch-off points from the structural units (II) is at most half the thickness (ii).

3. The composite according to claim 1 or 2, wherein a plurality of structural units (I), (II) and (III) and a plurality of branches of the structural units (II) branching off from (I) and (III) branching off from (II) are present.

4. The composite according to any of the preceding claims, wherein structural units (I), (II) and (III) are each independently planar.

5. The composite conforming to one of the preceding claims, the inner surface of which is at least twice as large as the outer surface.

6. The composite according to one of the preceding claims whose surface roughness Z_{q} determined by scanning force microscopy is 1 to 100 nm.

7. The composite according to one of the preceding claims, wherein the mean size D_{n,50} of the nanoparticles is selected from the range of 10 to 1000 nm.

8. The composite according to any of the preceding claims, wherein the matrix formed by the photostructured matrix material has a structure whose size is selected from the range of 0.1 to 100 µm, wherein the structural size of the matrix and the size of the nanoparticles are selected such that a plurality of nanoparticles may each be contained in the voids of the matrix.

9. The composite according to one of the preceding claims, wherein the nanoparticles are contained in a volume fraction of 10 to 60% and the average size of the nanoparticles is selected from the range of 30 to 600 nm.

10. A process for preparing a composite according to any one of claims 1 to 9 containing structured matrix material and nanoparticles contained therein comprising the steps of:
(a) a step in which a composite composition comprising a photostructurable material and nanoparticles is provided,
(b) a step in which at least one polymerization reaction is carried out by irradiating the photostructurable material to form a matrix to obtain the composite,
**characterized in that** the refractive index of the nanoparticle composite composition differs from the refractive index of the nanoparticle-free composite composition by less than 0.5 at the wavelength of irradiation performed in the at least one polymerization reaction in step (b).

11. The process according to claim 10, wherein step (b) comprises two polymerization reactions when structural units (I) and (II) are formed or three polymerization reactions when structural units (I), (II) and (III) are formed, wherein in each of the respective polymerization reactions a part of the photopolymerizable materials is reacted.

12. The method according to claim 11, wherein the two or three polymerization reactions are effected by the presence of different photostructurable materials and/or by variation of at least one process parameter selected from irradiation wavelength, irradiation power density and irradiation duration.

13. The process according to any one of claims 10 to 12, wherein structural units (I) are formed by a polymerization reaction and structural units (II) are formed by a further polymerization reaction and, if present, structural units (III) are formed by a further polymerization reaction.

14. Use of the composite according to any of claims 1 to 9 as a scaffold for biological cells.

## Revendications

1. Composite, qui peut être produit par la photostructuration d'un matériau de matrice photostructurable dans une préparation de composite contenant le matériau de matrice photostructurable et des nanoparticules dans une fraction volumique d'au moins 1 % en formant une matrice structurée avec des nanoparticules contenues dans celle-ci, l'indice de réfraction du composite avec des nanoparticules se distinguant de l'indice de réfraction du composite sans nanoparticules de moins de 0,5 pour une longueur d'onde choisie dans la plage allant de 150 nm à 2 000 nm, dans lequel le composite est élaboré de façon hiérarchisée et comprend au moins une unité structurelle (I) d'une épaisseur (i) choisie dans la plage allant de 10 µm à 100 mm et des unités structurelles (II) ramifiées sur l'unité structurelle (I) d'une épaisseur (ii) respectivement choisie dans la plage allant de 100 nm à 1 000 µm, l'épaisseur (ii) représentant au plus la moitié de l'épaisseur (i) au niveau des ramifications.

2. Composite selon la revendication 1, qui comprend au moins une unité structurelle (I) d'une épaisseur (i) choisie dans la plage allant de 100 µm à 50 mm, des unités structurelles (II) ramifiées sur l'unité structurelle (I) d'une épaisseur (ii) respectivement choisie dans la plage allant de 10 µm à 1 000 µm ainsi que des unités structurelles (III) ramifiées sur les unités structurelles (II) d'une épaisseur (iii) respectivement choisie dans la plage allant de 100 nm à 100 µm, l'épaisseur (ii) représentant au plus la moitié de l'épaisseur (i) au niveau des ramifications des unités structurelles (II) sur l'unité structurelle (I) et l'épaisseur (iii) représentant au plus la moitié de l'épaisseur (ii) au niveau des ramifications des unités structurelles (III) sur les unités structurelles (II).

3. Composite selon la revendication 1 ou 2, dans lequel une pluralité d'unités structurelles (I), (II) et (III) et une pluralité de ramifications des unités structurelles (II) sur (I) et (III) sur (II) sont présentes.

4. Composite selon une des revendications précédentes, dans lequel les unités structurelles (I), (II) et (III) sont planes de manière respectivement indépendante les unes des autres.

5. Composite selon une des revendications précédentes, dont la surface intérieure est au moins deux fois plus grande que la surface extérieure.

6. Composite selon une des revendications précédentes, dont la rugosité de surface Z_{q} définie par microscopie à force atomique représente 1 à 100 nm.

7. Composite selon une des revendications précédentes, dans lequel la taille moyenne D_{n,50} des nanoparticules est choisie dans la plage allant de 10 à 1 000 nm.

8. Composite selon une des revendications précédentes, dans lequel la matrice formée par le matériau de matrice photostructuré présente une structure dont la taille est choisie dans la plage allant de 0,1 à 100 µm, la taille de la structure de la matrice et la taille des nanoparticules étant choisies de telle sorte que les cavités de la matrice peuvent contenir respectivement une pluralité de nanoparticules.

9. Composite selon une des revendications précédentes, dans lequel les nanoparticules sont contenues dans une fraction volumique de 10 à 60 % et la taille moyenne des nanoparticules est choisie dans la plage allant de 30 à 600 nm.

10. Procédé destiné à produire un composite selon une des revendications 1 à 9, qui contient le matériau de matrice structuré et les nanoparticules contenues dans celui-ci, et qui comprend les étapes suivantes :
(a) une étape dans laquelle une préparation de composite contenant le matériau photostructurable et des nanoparticules est fournie,
(b) une étape dans laquelle au moins une réaction de polymérisation est réalisée sous rayonnement du matériau photostructurable en formant une matrice permettant d'obtenir le composite,
**caractérisé en ce que**, pour la longueur d'onde du rayonnement réalisé dans l'au moins une réaction de polymérisation à l'étape (b), l'indice de réfraction de la préparation de composite avec des nanoparticules se distingue de l'indice de réfraction de la préparation de composite sans nanoparticules de moins de 0,5.

11. Procédé selon la revendication 10, dans lequel l'étape (b) comprend deux réactions de polymérisation lorsque des unités structurelles (I) et (II) sont formées, ou trois réactions de polymérisation lorsque des unités structurelles (I), (II) et (III) sont formées, une partie des matériaux photopolymérisables étant respectivement transférée dans les réactions de polymérisation respectives.

12. Procédé selon la revendication 11, dans lequel les deux ou trois réactions de polymérisation sont obtenues en présence de différents matériaux photostructurables et/ou en modifiant au moins un paramètre de procédé qui est choisi parmi la longueur d'onde du rayonnement, la densité de puissance du rayonnement et la durée du rayonnement.

13. Procédé selon une des revendications 10 à 12, dans lequel les unités structurelles (I) sont formées par une réaction de polymérisation et les unités structurelles (II) sont formées par une autre réaction de polymérisation ainsi que, si elles sont présentes, les unités structurelles (III) sont formées par une autre réaction de polymérisation.

14. Utilisation du composite selon une des revendications 1 à 9 en tant que cadre pour des cellules biologiques.
